(19) 

**Europäisches Patentamt**
**European Patent Office**
**Office européen des brevets**

(11) **EP 4 653 468 A1**

(12) **EUROPEAN PATENT APPLICATION**
published in accordance with Art. 153(4) EPC

(43) Date of publication:
26.11.2025 Bulletin 2025/48

(21) Application number: 24744226.2

(22) Date of filing: 16.01.2024

(51) International Patent Classification (IPC):
*C07K 19/00* (2006.01)      *C12N 15/63* (2006.01)
*C12N 5/10* (2006.01)       *C12N 15/11* (2006.01)
*A61K 38/16* (2006.01)      *A61K 48/00* (2006.01)
*A61P 9/10* (2006.01)

(52) Cooperative Patent Classification (CPC):
A61K 38/16; A61K 48/00; A61P 9/10; C07K 19/00;
C12N 5/10; C12N 15/11; C12N 15/63

(86) International application number:
PCT/CN2024/072443

(87) International publication number:
WO 2024/153050 (25.07.2024 Gazette 2024/30)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 17.01.2023 CN 202310059450

(71) Applicant: Suzhou Youthen Biotechnology Co.,
Ltd
Suzhou, Jiangsu 215123 (CN)

(72) Inventors:
• MA, Yuanfang
Suzhou, Jiangsu 215124 (CN)

• WEI, Yinxiang
Suzhou, Jiangsu 215124 (CN)
• ZHANG, Hailong
Suzhou, Jiangsu 215124 (CN)

(74) Representative: karo IP
Patentanwälte PartG mbB
Steinstraße 16-18
40212 Düsseldorf (DE)

Remarks:
The complete document including Reference
Table(s) and the Sequence Listing(s) can be
downloaded from the EPO website

(54) **DR5 DOMAIN VARIANT AND USE THEREOF**

(57) The present application relates to a death receptor 5 (DR5) domain variant. The present application also relates to a fusion protein containing the DR5 domain variant. The fusion protein has one or more of the following properties: (1) being capable of binding to TRAIL; (2) blocking TRAIL/DR5 pathway-induced apoptosis; (3) reducing the area of myocardial infarction in cardiac ischemia and/or ischemia-reperfusion; and (4) having protective effects against cerebral infarction. The present application further provides a method for preparing the fusion protein, and the use thereof.

EP 4 653 468 A1

**Description**

**TECHNICAL FIELD**

The present application relates to the field of biomedicine, in particular to a DR5 domain variant, a fusion protein containing the DR5 domain variant, and use thereof.

**BACKGROUND**

Acute myocardial infarction (AMI) is a serious cardiovascular emergency. The incidence of myocardial infarction in China is about 50 cases per100,000 people, with more than 600,000 new cases each year and more than 1 million people dying from myocardial infarction and its complications. With the aggravated aging of the population, the incidence of AMI has been rising year by year and is showing a trend towards younger people.

After myocardial infarction is occurred, cardiomyocytes die at a rate of 20% per hour. Therefore, time is myocardium, and time is life. With the development of medical technology, patients suffering from acute myocardial infarction can receive thrombolysis or percutaneous coronary intervention (PCI, i.e., stent placement) in the early stage of the disease to restore blood supply, and thus reduce the area of myocardial infarction. However, the "golden rescue time" for PCI is only 120 minutes (the mortality rate is about 5% if the PCI is completed within 120 minutes, and the mortality rate of PCI reaches up to 10% if the PCI is completed within 240 minutes). The number of patients who can complete the PCI within the "golden rescue time" is less than 5% all over the world. In addition, the recanalization of blood flow will cause myocardial cells to experience a second injury, namely "reperfusion injury", which will cause the death of more cells and even endanger life. At the same time, reperfusion injury can also lead to late ventricular remodeling and heart failure, seriously affecting the patient's quality of life. At present, there is no specific therapeutic drugs against myocardial cell ischemia and ischemia-reperfusion injury in clinic all over the world.

Cell death includes necrosis and apoptosis, of which necrosis was once considered the dominant factor for myocardial infarction. In recent years, the role of apoptosis in myocardial infarction has gotten more and more attention. The death receptor pathway activated by the binding of death receptor 5 (DR5) to tumor necrosis factor (TNF)-related apoptosis-inducing ligand (TRAIL) can recruit related proteins to form a death-inducing signaling complex (DISC), which in turn activates Caspase-8, Caspase-3, etc. in cascade, thereby causing apoptosis. Soluble death receptor 5 (soluble DR5 or sDR5) is expressed at a low level in the peripheral blood of health people. Because it has a complete extracellular segment structure that binds to the TRAIL ligand, it can compete with the death receptor on the cell membrane to bind to the TRAIL molecule, thereby blocking the TRAIL-induced apoptosis.

The existing DR5 fusion protein still has some problems in the treatment of myocardial infarction, such as high dosage required, limited administration route, etc. Therefore, there is a need to develop novel DR5 fusion proteins to solve such problems.

**SUMMARY OF THE INVENTION**

The present application provides a death receptor 5 (DR5) domain variant and a fusion protein containing the DR5 domain variant, which have one or more of the following properties: (1) being capable of binding to TRAIL at a relatively high affinity; (2) blocking TRAIL/DR5 pathway-induced apoptosis; (3) reducing the area of myocardial infarction in cardiac ischemia and/or ischemia-reperfusion; and (4) having protective effects against cerebral infarction. The fusion protein described in the present application can achieve specific protective effects against myocardial infarction and/or cerebral infarction at a relatively low dose. The fusion protein described in the present application can also effectively extend the half-life of a drug in vivo, and can solve the problem of frequent drug administration. The fusion protein described in the present application can exert protective effects with only a relatively small dose required, and can be administered intracranially or injected intramuscularly.

In one aspect, the present application provides a death receptor 5 (DR5) domain variant.

In some embodiments, the DR5 domain comprises an extracellular domain of the DR5.

In some embodiments, the DR5 domain variant comprises an amino acid mutation(s) at one or more amino acid sites from positions 86 to 155 compared to the extracellular domain of human DR5 variant 1 (SEQ ID NO: 94).

In some embodiments, the death receptor 5 (DR5) includes soluble death receptor 5 (sDR5).

In some embodiments, the DR5 is human DR5.

In some embodiments, the DR5 domain variant comprises an amino acid mutation(s) at one or more amino acid sites from positions 86 to 92.

In some embodiments, the DR5 domain variant comprises an amino acid mutation(s) at one or more amino acid sites from positions 105 to 112.

In some embodiments, the DR5 domain variant comprises an amino acid mutation(s) at one or both amino acid sites from positions 114 and 155.

In some embodiments, the DR5 domain variant comprises an amino acid mutation(s) at one or both amino acid sites from positions 114 and 115.

In some embodiments, the DR5 domain variant comprises an amino acid mutation(s) at one or more amino acid sites from positions 147 to 152.

In some embodiments, the DR5 domain variant comprises an amino acid mutation(s) at one or more amino acid sites from positions 154 to 155.

In some embodiments, the DR5 domain variant comprises an amino acid mutation(s) at one or more amino acid sites selected from the group consisting of I87, E89, D90, T105, N108, D109, L114, R115, E147, D148, E151, R154, and K155 compared to the amino acid sequence as set forth in SEQ ID NO: 94.

In some embodiments, the DR5 domain variant comprises a mutation(s) at the amino acid site(s) selected from any one of the following groups, compared to the amino acid sequence as set forth in SEQ ID NO: 94:

(1) L114 and R115;
(2) R154 and K155;
(3) I87, D90, T105, D109, and D148;
(4) I87, D90, T105, D109, L114, E147;
(5) I87, E89, D90, D109, E147;
(6) I87, E89, D90, T105, D109, L114, D148;
(7) D90, T105, D109, L114, D148, R154;
(8) D90, D109, and D148;
(9) I87, D90, and R154;
(10) T105, N108, and F112;
(11) L114, E147, and E151; and
(12) R115.

In some embodiments, in the amino acid mutations, the mutated amino acid is a polar amino acid.

In some embodiments, in the amino acid mutations, the mutated amino acid is a neutral amino acid.

In some embodiments, in the amino acid mutations, the mutated amino acid is an acidic amino acid.

In some embodiments, in the amino acid mutations, the mutated amino acid is a basic amino acid.

In some embodiments, in the amino acid mutations, the mutated amino acid is a non-polar amino acid.

In some embodiments, in the amino acid mutations, the mutated amino acid is an amino acid with a relatively small side chain volume.

In some embodiments, the DR5 domain variant comprises one or more amino acid mutations selected from the group consisting of I87A/V/L, E89N/Q, D90N/S, T105N/S, N108Q, D109H/S/Q/E, F112Y, L114A/G/V/S/W/Y, R115A/G/V/L/S/W/Y, E147S/Y, D148N/K/E, E151S, R154A/G/V/L/S/W/Y, and K155A/G/V/L/S/W/Y, compared to the amino acid sequence as set forth in SEQ ID NO: 94.

In some embodiments, the DR5 domain variant comprises the amino acid mutation(s) selected from any one of the following groups, compared to the amino acid sequence as set forth in SEQ ID NO: 94:

(1) L114A and R115A;
(2) R154A and K155A;
(3) I87L, D90S, T105N, D109H, and D148N;
(4) I87A, D90S, T105N, D109S, L114V, and E147S;
(5) I87A, E89N, D90N, D109Q, and E147Y;
(6) I87A, E89Q, D90S, T105S, D109S, L114V, and D148K;
(7) D90S, T105N, D109E, L114I, D148E, and R154Y;
(8) D90S, D109N, and D148E;
(9) I87V, D90N, and R154Y;
(10) T105S, N108Q, and F112Y;
(11) L114V, E147Q, and E151S;
(12) L114G and R115G;
(13) L114V and R115V;
(14) R115L;
(15) L114S and R115S;
(16) L114W and R115W;
(17) L114Y and R115Y;
(18) R154G and K155G;

(19) R154V and K155V;
(20) R154L and K155L;
(21) R154S and K155S;
(22) R154W and K155W; and
(23) R154Y and K155Y.

In some embodiments, the DR5 domain variant comprises the amino acid sequence as set forth in any one of SEQ ID NOs: 1-23.

In another aspect, the present application provides a fusion protein comprising the DR5 domain variant.

In some embodiments, the fusion protein further comprises the immunoglobulin Fc region.

In some embodiments, the immunoglobulin Fc region comprises the Fc region of IgG.

In some embodiments, the IgG is selected from the group consisting of IgG1 and IgG4.

In some embodiments, the immunoglobulin Fc region comprises the amino acid sequence as set forth in any one of SEQ ID NO: 97, SEQ ID NO: 98, and SEQ ID NO: 99.

In some embodiments, the DR5 domain variant in the fusion protein is located at the N-terminus of the immunoglobulin Fc region.

In some embodiments, the DR5 domain variant in the fusion protein is located at the C-terminus of the immunoglobulin Fc region.

In some embodiments, the fusion protein further comprises a protein capable of extending half-life.

In some embodiments, the protein capable of extending half-life comprises human serum albumin.

In some embodiments, the protein capable of extending half-life in the fusion protein is located at the N-terminus of the DR5 domain variant.

In some embodiments, the protein capable of extending half-life in the fusion protein is located at the C-terminus of the DR5 domain variant.

In some embodiments, the fusion protein comprises the amino acid sequence as set forth in any one of SEQ ID NOs: 24-92.

In another aspect, the present application further provides one or more isolated nucleic acid molecules encoding the DR5 domain variant or the fusion protein.

In another aspect, the present application further provides a vector, comprising the nucleic acid molecule.

In another aspect, the present application further provides a cell, comprising the nucleic acid molecule or the vector.

In another aspect, the present application further provides a method for preparing the DR5 domain variant or the fusion protein, comprising culturing the cell under conditions that allow the expression of the DR5 domain variant or the fusion protein.

In another aspect, the present application further provides a pharmaceutical composition comprising the DR5 domain variant, the fusion protein, the nucleic acid molecule, the vector and/or the cell, and optionally a pharmaceutically acceptable carrier.

In some embodiments, the pharmaceutical composition further comprises other active ingredients.

In another aspect, the present application further provides a method for preventing and/or treating a disease and/or disorder, comprising administering the DR5 domain variant, the fusion protein, the nucleic acid molecule, the vector, the cell, and/or the pharmaceutical composition to a subject in need thereof.

In some embodiments, the disease and/or disorder includes myocardial infarction and/or myocardial infarction-reperfusion injury.

In some embodiments, the disease and/or disorder includes cerebral infarction.

In another aspect, the present application further provides use of the DR5 domain variant, the fusion protein, the nucleic acid molecule, the vector, the cell, and/or the pharmaceutical composition in preparation of a medicament which is used for preventing and/or treating a disease and/or disorder.

In some embodiments, the disease and/or disorder includes myocardial infarction and/or myocardial infarction-reperfusion injury.

In some embodiments, the disease and/or disorder includes cerebral infarction.

In another aspect, the present application further provides the DR5 domain variant, the fusion protein, the nucleic acid molecule, the vector, the cell, and/or the pharmaceutical composition, for use in preventing and/or treating diseases and/or disorders.

In some embodiments, the disease and/or disorder includes myocardial infarction and/or myocardial infarction-reperfusion injury.

In some embodiments, the disease and/or disorder includes cerebral infarction.

In another aspect, the present application further provides a method for blocking the TRAIL/DR5 pathway, comprising administering the fusion protein or the pharmaceutical composition.

Those skilled in the art may easily discern other aspects and advantages of the present application from the following detailed description. Only exemplary embodiments of the present application are shown and described in the following detailed description. As those skilled in the art will realize, the contents of the present application enable those skilled in the art to make modifications to the specific embodiments disclosed without departing from the spirit and scope of the invention to which the present application relates. Accordingly, the drawings and descriptions in the specification of the present application are illustrative only and not limited.

## BRIEF DESCRIPTION OF DRAWINGS

The specific features of the invention to which the present application relates are as shown in the appended claims. The features and advantages of the invention to which the present application relates may be better understood by reference to the exemplary embodiments and the drawings described in detail below. A brief description of the drawings is as follows:

Fig. 1 shows a schematic diagram of the physical structure of vector pTM.

Fig. 2 shows a schematic diagram of a method for detecting the interaction between DR5 truncated domains and mutants thereof and TRAIL.

Fig. 3 shows the results of enriching and screening by flow cytometry of the DR5 truncated domain mutants.

Fig. 4 shows the circumstances of sequence alignment of the DR5 truncated domains and variants thereof.

Fig. 5 shows the circumstances of recognition of the TRAIL protein by the fusion protein of the present application.

Fig. 6 shows the activity of the fusion protein of the present application in blocking the TRAIL-induced apoptosis.

Fig. 7 shows the activity of the fusion protein of the present application in alleviating the cardiac ischemia/reperfusion injury in rats.

Fig. 8 shows the activity of the fusion protein of the present application in alleviating the cardiac ischemia/reperfusion injury in rhesus monkeys.

Fig. 9 shows the activity of the fusion protein of the present application in blocking the TRAIL-induced apoptosis.

Fig. 10 shows the activity of the fusion protein of the present application in alleviating the cardiac ischemia/reperfusion injury in rats.

Fig. 11 shows the activity of the fusion protein of the present application in alleviating the cerebral ischemia/reperfusion injury in rats.

Fig. 12 shows the activity of the fusion protein of the present application by intramuscular injection in protecting from myocardial infarction/reperfusion injury.

## DETAILED DESCRIPTION

The embodiments of the invention of the present application will be illustrated below with specific examples. Those skilled in the art may easily understand other advantages and effects of the invention of the present application from the disclosure of the specification.

## Definition of Terms

In the present application, the term "fusion protein" generally refers to a protein composed of two or more polypeptides. Although the polypeptides are not typically bound in native state, they are bound together by peptide bonds at respective amino- and carboxyl-termini to form a continuous polypeptide. It should be understood that the two or more peptide components can bind directly or indirectly via peptide linkers/spacers. Fusion proteins can generally be artificially prepared using methods such as nucleic acid recombination or chemical synthesis.

In the present application, the term "DR5" is also called death receptor 5 (DR5). The death receptor pathway activated by the binding of the DR5 to tumor necrosis factor (TNF)-related apoptosis-inducing ligand (TRAIL) can recruit related proteins to form a death-inducing signaling complex (DISC), which in turn activates Caspase-8, Caspase-3, etc. in cascade, thereby causing apoptosis. In the present application, the DR5 can be human DR5. In the present application, DR5 can be soluble DR5 (sDR5). The term also encompasses full-length DR5, functionally active fragments thereof, variants thereof, congeners thereof, or analogs thereof.

In the present application, the term "DR5 domain" generally refers to wild-type DR5, a fragment or a functional variant thereof. For example, the DR5 domain can comprise the ectodomain of DR5. For example, the ectodomain of human DR5 variant 1 can comprise an amino acid sequence as set forth in SEQ ID NO: 94. For example, the DR5 domain can comprise one or more amino acid mutations compared to the corresponding human DR5 variant 1 or a fragment thereof.

In the present application, the term "immunoglobulin Fc region" generally refers to the base region of the Y-shaped structure of the antibody structure, also known as the Fragment crystallizable region (Fc region). In the IgG, IgA, and IgD antibody isotypes, the Fc region consists of two identical protein fragments derived from the second and third constant domains of the two heavy chains of the antibody; and the Fc regions of IgM and IgE contain three heavy chain constant domains in each polypeptide chain. For example, the immunoglobulin Fc region described in the present application may comprise the Fc region of IgG, IgA, IgD, IgM, or IgE. In some embodiments, the immunoglobulin Fc region may comprise the Fc region of IgG. For example, the immunoglobulin Fc region may comprise the Fc region of IgG1. For example, the immunoglobulin Fc region may comprise the Fc region of IgG4.

In the present application, the term "IgG" generally refers to Immunoglobulin G. IgG is one of human immunoglobulins, and the others are IgA, IgM, IgD, and IgE. According to the antigenic differences of the gamma chain in IgG molecules, there are four subtypes for human IgG: IgG1, IgG2, IgG3, and IgG4. In the present application, the term "IgG1" generally refers to a subtype with the highest proportion in IgG, which has a relatively high affinity to Fc receptors. For example, the IgG may be human IgG. Again by the way of example, the IgG can be IgG1.

In the present application, the DR5 domain may comprise the human DRR5 domain, and a fragment or a variant thereof. For example, the DR5 domain may comprise the ectodomain of DR5, and a fragment or a variant thereof.

In the present application, the term "extracellular domain" can be used interchangeably with "ectodomain", which generally refers to the functional structure region of a protein located outside the cell membrane. In some embodiments, the ectodomain refers to the ectodomain of the DR5 domain, a fragment or a variant thereof. For example, the ectodomain of the human DR5 variant 1 may comprise an amino acid sequence as set forth in SEQ ID NO: 94.

The DR5 domain variant described in the present application may comprise an amino acid sequence having at least 80% (e.g., at least 85%, at least 90%, at least 91%, at least 92%, at least 93%, at least 94%, at least 95%, at least 96%, at least 97%, at least 98%, at least 99%, or at least 100%) sequence homology to the amino acid sequence as set forth in any one of SEQ ID NOs: 1-23.

The fusion protein described in the present application may comprise an amino acid sequence having at least 80% (e.g., at least 85%, at least 90%, at least 91%, at least 92%, at least 93%, at least 94%, at least 95%, at least 96%, at least 97%, at least 98%, at least 99%, or at least 100%) sequence homology to the amino acid sequence as set forth in any one of SEQ ID NOs: 24-92.

In the present application, the term "sequence homology" generally refers to sequence similarity, interchangeability, analogy, or correlation between two or more polynucleotide sequences or between two or more polypeptide sequences. When using a computer program or software (e.g., Emboss Needle or BestFit) to determine sequence identity, similarity, or homology between different amino acid sequences, default parameter settings may be used. An appropriate scoring matrix, such as blosum45 or blosum80, can also be selected to optimize identity, similarity, or homology scores. In some embodiments, homologous polynucleotides include the polynucleotides that are capable of hybridizing to a control polynucleotide sequence under stringent conditions and have at least 60%, at least 65%, at least 70%, at least 80%, at least 90%, at least 95%, at least 97%, at least 98%, at least 99% or even at least 100% sequence identity to the control polynucleotide sequence. Homologous polypeptides may be the polypeptides that have at least 80%, at least 85%, at least 90%, at least 95%, at least 97%, at least 98%, at least 99%, or even at least 100% sequence identity to a control polypeptide sequence when the sequences are aligned under optimized conditions.

In the present application, the "percentage of sequence homology" can be calculated by comparing two sequences to be aligned in a comparison window, determining the number of sites in which the same nucleic acid bases (e.g., A, T, C, G, and I) or the same amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are present in the two sequences to obtain the number of matching sites, dividing the number of matching sites by the total number of sites in the comparison window (i.e., window size), and multiplying the result by 100 to produce the percentage of sequence homology. Alignment for the purpose of determining percent of sequence homology can be accomplished in a variety of ways known in the art, for example, using publicly available computer software such as BLAST, BLAST-2, ALIGN, or Megalign (DNASTAR) software. Those skilled in the art can determine appropriate parameters for aligning sequences, including any algorithm required to achieve maximal alignment within the range of full-length sequences being compared or within the sequence region of interest. The homology can also be determined by the following methods: FASTA and BLAST. A description of the FASTA algorithm can be found in W. R. Pearson and D. J. Lipman, "Improved Tool for Biological Sequence Comparison", (Proc. Natl. Acad. Sci. USA), 85: 2444-2448, 1988; and D. J. Lipman and W. R. Pearson, "Fast and Sensitive Protein Similarity Search", Science, 227: 1435-1441, 1989. A description of the BLAST algorithm can be found in S. Altschul, W. Gish, W. Miller, E. W. Myers and D. Lipman, "A Basic Local Alignment Search Tool", Molecular Biology, 215: 403-410, 1990.

In the present application, an amino acid sequence (e.g., a protein domain or a protein fragment, such as the DR5 domain variant described in the present application, or the fusion protein described in the present application) may comprise the substitution(s), deletion(s) or addition(s) of one or more amino acid residues. In the present application, for example the DR5 domain variant may comprise an amino acid mutation(s) at one or more residues selected from the group consisting of: I87, E89, D90, T105, N108, D109, L114, R115, E147, D148, E151, R154, and K155.

In the present application, the position of the amino acid residue in the amino acid mutations is numbered by the residue determined based on the amino acid sequence as set forth in SEQ ID NO: 96 (the extracellular domain of human DR5 variant 1 (containing signal peptide)).

In the present application, "amino acid site Xn" typically refers to residue X corresponding to the position n of the amino acid sequence as set forth in SEQ ID NO: 96, where n is a positive integer and X is an abbreviation for any amino acid residue. For example, "residue I87" represents the amino acid residue I corresponding to the position 87 of the amino acid sequence as set forth in SEQ ID NO: 96.

In the present application, the amino acid sequence as set forth in SEQ ID NO: 94 is the positions 57 to 182 of the amino acid sites as set forth in SEQ ID NO: 96. The position of the amino acid sequences of the present application is determined based on SEQ ID NO: 96, that is, the numbering of the amino acid sequence as set forth in SEQ ID NO: 94 is from I57 to E182, and the numbering of the first amino acid of the amino acid sequence as set forth in SEQ ID NO: 94 is I57.

In the present application, a certain residue in a certain amino acid sequence "corresponding to" a certain residue in another amino acid sequence generally refers to the residue correspondence obtained when the amino acid sequences are aligned under optimized conditions. The sequence alignment can be performed in a manner understood by those skilled in the art, for example, using BLAST, BLAST-2, ALIGN, NEEDLE, or Megalign (DNASTAR) software. Those skilled in the art will be able to determine appropriate parameters for alignment, including any algorithm required to achieve optimal alignment across the full-length sequences being compared.

The amino acid substitution described in the present application may be a non-conservative substitution. The non-conservative substitution may include changing an amino acid residue in the target protein or polypeptide in a non-conservative manner, for example, changing an amino acid residue with a certain side chain size or a certain property (for example, hydrophilicity) to an amino acid residue with a different side chain size or different property (e.g., hydrophobicity).

The amino acid substitution may also be a conservative substitution. The conservative substitution may include changing an amino acid residue in the target protein or polypeptide in a conservative manner, for example, changing an amino acid residue with a certain side chain size or a certain property (for example, hydrophilicity) to an amino acid residue with an identical or similar side chain size or identical or similar property (e.g., still hydrophilicity). Such conservative substitutions generally do not have a significant impact on the structure or function of the resulting protein. In the present application, amino acid sequence variants as the fusion proteins or fragments thereof may include conservative amino acid substitutions that do not significantly change the protein's structure or function (e.g., the ability to block the TRAIL/DR5 pathway).

In the present application, the amino acid mutation "XnY/Z" means that the residue X at the position n corresponding to the amino acid sequence as set forth in SEQ ID NO: 96 is substituted with the amino acid residue Y or the amino acid residue Z, where n is a positive integer, and X, Y, and Z are each independently an abbreviation of any amino acid residue, and X is different from Y and Z. For example, the amino acid substitution "L114A/G/V/ S/W/Y" means that the residue L at position 114 corresponding to the amino acid sequence as set forth in SEQ ID NO: 96 is substituted with the amino acid residue A, G, V, S, W, or Y.

In the present application, the term "amino acid mutations" typically mean to encompass amino acid substitutions, deletions, insertions, and modifications. Any combination of substitutions, deletions, insertions, and modifications can be made to achieve the final construct, provided that the final construct possesses the desired properties. Deletions and insertions of an amino acid sequence include deletions at the amino- and/or carboxyl-terminus and amino acid insertions. Particular amino acid mutations are amino acid substitutions. For example, one amino acid is replaced with another amino acid having different structural and/or chemical properties. Amino acid substitutions include replacement with non-naturally occurring amino acids or with naturally occurring amino acid derivatives of the 20 standard amino acids (e.g., 4-hydroxyproline, 3-methylhistidine, ornithine, homoserine, and 5-hydroxylysine). Amino acid mutations can be generated using genetic or chemical methods well known in the art. Genetic methods can include site-directed mutagenesis, PCR, gene synthesis, etc. A method of changing the amino acid side chain groups by methods other than genetic engineering such as chemical modification is also available.

In the present application, the term "nucleic acid molecule" generally refers to any length of isolated nucleotides, deoxyribonucleotides or ribonucleotides or analogs thereof that are isolated from their natural environment or are artificially synthesized. The nucleic acid molecule according to the present application can be isolated. For example, it can be produced or synthesized by: (i) amplification in vitro, such as amplification by polymerase chain reaction (PCR), (ii) clonal recombination, (iii) purification, such as by enzymatic digestion and gel electrophoresis fractionation, or (iv) synthesis, such as chemical synthesis. In some embodiments, the isolated nucleic acid is a nucleic acid molecule prepared by recombinant DNA technology. In the present application, nucleic acids encoding the antibodies or antigen-binding fragments thereof can be prepared by a variety of methods known in the art, including but not limited to, overlap extension PCR using restricted fragment manipulation or using synthetic oligonucleotides. For specific operations, see Sambrook et al., Molecular Cloning, A Laboratory Manual, Cold Spring Harbor Laboratory Press, Cold Spring Harbor, N.Y., 1989; and Ausube et al., Current Protocols in Molecular Biology, Greene Publishing and Wiley-Interscience, New York N.Y., 1993.

In the present application, the term "vector" generally refers to a nucleic acid molecule that is capable of self-replicating in a suitable host, transferring the inserted nucleic acid molecule to and/or between host cells. The vectors may include vectors primarily used for insertion of DNAs or RNAs into cells, vectors primarily used for replication of DNAs or RNAs, and vectors primarily used for expression, transcription and/or translation of DNAs or RNAs. The vectors also include vectors having a variety of the above-mentioned functions. The vectors may be polynucleotides capable of being transcribed and translated into a polypeptide when introduced into suitable host cells. Generally, the vectors can produce the desired expression products by culturing appropriate host cells comprising the vectors. In the present application, one or more of the nucleic acid molecules may be comprised in the vectors. In addition, the vectors may further comprise other genes, such as marker genes which allow the vectors to be selected in suitable host cells and under suitable conditions. In addition, the vectors may further comprise expression control elements that allow correct expression of coding regions in an appropriate host. Such control elements are well known to those skilled in the art, and may include, for example, a promoter, a ribosome binding site, an enhancer, and other control elements that regulate gene transcription or mRNA translation. In some embodiments, the expression control sequences are regulatable elements. The specific structures of the expression control sequences may vary depending on species or the function of cell types, but generally include a 5' non-transcribed sequence and 5' and 3' non-translated sequences involved in the initiation of transcription and translation, respectively, for example, a TATA box, a caped sequence, and a CAAT sequence. For example, the 5' non-transcribed expression control sequence may comprise a promoter region, and the promoter region may comprise a promoter sequence for transcriptionally controlling the functionally linked nucleic acid. In addition, the vector may also include a replication initiation site. The vector may also include components that facilitate to enter cells, such as viral particles, liposomes, or protein coats, which are not limited thereto.

In the present application, the term "host cell", "cell", and "host" may be used interchangeably, and generally refer to individual cells, cell lines or cell cultures which may contain or have contained plasmids or vectors comprising the nucleic acid molecules described in the present application, or which are capable of expressing the fusion proteins, fragments or variants thereof described in the present application. The host cells may include progeny of a single host cell. Due to natural, accidental or intentional mutations, the progeny cells may not necessarily be completely identical in morphology or genome to the original parent cells, but they can express the antibodies or antigen-binding fragments thereof described in the present application. The host cells can be obtained by transfecting cells in vitro using the vectors described in the present application. The host cells can be prokaryotic cells (such as E. coli) or eukaryotic cells (such as yeast cells, such as COS cells, Chinese hamster ovary (CHO) cells, HeLa cells, HEK293 cells, COS-1 cells, NS0 cells or myeloma cells).

In the present application, the term "pharmaceutical composition" typically refers to a composition for use in prevention/treatment of diseases or disorders. The pharmaceutical composition can comprise the fusion protein, the nucleic acid molecule, the vector, and/or the cell described in the present application, and optionally a pharmaceutically acceptable carrier. In addition, the pharmaceutical composition can further comprise suitable preparations of one or more (pharmaceutically effective) adjuvants, stabilizers, excipients, diluents, solubilizers, surfactants, emulsifiers, and/or preservatives. The acceptable components of the composition are preferably non-toxic to a recipient at the dose and concentration used. Pharmaceutical compositions of the present invention include, but are not limited to, liquid, frozen, and lyophilized compositions.

In the present application, the term "pharmaceutically acceptable carrier" generally includes pharmaceutically acceptable carriers, excipients or stabilizers that are non-toxic to the cells or mammals to which they are exposed at the doses and concentrations used. Physiologically acceptable carriers may include, for example, buffers, antioxidants, polypeptides with low molecular weight (less than about 10 residues), proteins, hydrophilic polymers, amino acids, monosaccharides, disaccharides and other carbohydrates, chelating agents, sugar alcohols, salt-forming counter-ions such as sodium, and/or nonionic surfactants.

In the present application, the term "subject" generally refers to any human or non-human animal. The term "non-human animal" may include all vertebrates, such as mammals and non-mammals, such as non-human primates, goats, sheep, dogs, cattle, chickens, amphibians, reptiles, etc.

In the present application, the term "about" typically refers to a change within a range of 0.5%-10% greater than or less than a specified value, e.g., a change within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% greater than or less than the specified value.

In the present application, the term "including" generally refers to the meaning of comprising, covering, containing, or encompassing. In some cases, it also means "being", "consisting of".

## Detailed Description of the Invention

In one aspect, the present application provides a death receptor 5 (DR5) domain variant. The DR5 domain variant is capable of binding to TRAIL.

In the present application, the DR5 domain variant can block the TRAIL/DR5 signaling pathway, thereby blocking apoptosis induced by the TRAIL/DR5 pathway. In the present application, the DR5 domain variant can form a fusion protein with other fragments, and the fusion protein can reduce the area of myocardial infarction in cardiac ischemia. In the present application, the fusion protein can reduce the area of myocardial infarction in cardiac ischemia-reperfusion. In the present application, the fusion protein can have protective effects against cerebral infarction.

In the present application, the DR5 domain can comprise a functional active fragment of DR5, or a variant thereof. For example, the DR5 domain may comprise the extracellular domain of the DR5. For example, the extracellular domain of the DR5 may comprise a variant of the DR5 extracellular domain, and the variant can be obtained by performing amino acid mutations on the basis of the extracellular domain of human DR5 variant 1. For example, the extracellular domain of the human DR5 variant 1 may comprise the amino acid sequence as set forth in SEQ ID NO: 94. For example, the variant of the DR5 ectodomain may have at least 80% (e.g., at least 85%, at least 90%, at least 91%, at least 92%, at least 93%, at least 94%, at least 95%, at least 96%, at least 97%, at least 98%, at least 99%, or at least 100%) sequence homology to the extracellular domain of the human DR5 variant 1. In the present application, the DR5 can comprise human DR5. For example, the variant of the DR5 ectodomain may have 1, 2, 3, 4, 5, 6, 7, 8, 9, 10, 11, 12, 13, 14, 15, 16, 17, 18, 19, 20, 21, 22, 23, 24, or more amino acid mutations, compared to the wild type DR5 ectodomain. In the present application, the DR5 domain can be subjected to one or more amino acid mutations based on the extracellular domain of the human DR5 variant 1.

In the present application, the DR5 domain variant can comprise an amino acid mutation(s) at one or more amino acid sites from positions 86 to 92.

In the present application, the DR5 domain variant can comprise an amino acid mutation(s) at one or more amino acid sites from positions 105 to 1122.

In the present application, the DR5 domain variant can comprise an amino acid mutation(s) at one or more amino acid sites from positions 114 to 155.

In the present application, the DR5 domain variant can comprise an amino acid mutation(s) at one or more amino acid sites from positions 114 to 115.

In the present application, the DR5 domain variant can comprise an amino acid mutation(s) at one or more amino acid sites from positions 147 to 152.

In the present application, the DR5 domain variant can comprise an amino acid mutation(s) at one or more amino acid sites from positions 154 to 155.

For example, the DR5 domain in the fusion protein can comprise an amino acid mutation(s) at one or more amino acid sites selected from the group consisting of: I87, E89, D90, T105, N108, D109, L114, R115, E147, D148, E151, R154, and K155.

For example, the DR5 domain variant can comprise mutations at the amino acid sites L114 and R115. For example, the DR5 domain variant can comprise mutations at the amino acid sites R154 and K155. For example, the DR5 domain variant can comprise mutations at the amino acid sites I87, D90, T105, D109, and D148. For example, the DR5 domain variant can comprise mutations at the amino acid sites I87, D90, T105, D109, L114, and E147. For example, the DR5 domain variant can comprise mutations at the amino acid sites I87, E89, D90, D109, and E147. For example, the DR5 domain variant can comprise mutations at the amino acid sites I87, E89, D90, T105, D109, L114, and D148. For example, the DR5 domain variant can comprise mutations at the amino acid sites D90, T105, D109, L114, D148, and R154. For example, the DR5 domain variant can comprise mutations at the amino acid sites D90, D109, and D148. For example, the DR5 domain variant can comprise mutations at the amino acid sites I87, D90, and R154. For example, the DR5 domain variant can comprise mutations at the amino acid sites T105, N108, and F112. For example, the DR5 domain variant can comprise mutations at the amino acid sites L114, E147, and E151. For example, the DR5 domain variant can comprise a mutation at the amino acid site R115.

In the present application, at the site having the amino acid mutation, the mutated amino acid can be a non-polar amino acid. In the present application, at the site having the amino acid mutation, the mutated amino acid can be a polar amino acid. In the present application, at the site having the amino acid mutation, the mutated amino acid can be a neutral amino acid.

In the present application, at the site having the amino acid mutation, the mutated amino acid can be a hydrophilic amino acid. In the present application, at the site having the amino acid mutation, the mutated amino acid can be a hydrophobic amino acid.

In the present application, at the site having the amino acid mutation, the mutated amino acid can be an amino acid with a relatively small side chain volume. For example, the amino acid with a relatively small side chain volume is characterized in that the side chain is a hydrogen atom, methyl, or ethyl, which is a radical with a small volume.

For example, the amino acid group with non-polar side chains may include: alanine, valine, leucine, isoleucine, proline, phenylalanine, tryptophan, and methionine.

For example, the amino acid group with polar side chains may include: glycine, serine, threonine, cysteine, tyrosine, asparagine, glutamine, aspartic acid, glutamic acid, lysine, arginine, and histidine.

In the present application, in each amino acid mutation site, the mutated amino acid can be independently selected from: alanine (A), glycine (G), valine (V), leucine (L), serine (S), tryptophan (W), and tyrosine (Y).

In the present application, the DR5 domain variant may comprise one or more amino acid mutations selected from the group consisting of I87A/V/L, E89N/Q, D90N/S, T105N/S, N108Q, D109H/S/Q/E, F112Y, L114A/G/V/S/W/Y, R115A/G/V/L/S/W/Y, E147S/Y, D148N/K/E, E151S, R154A/G/V/L/S/W/Y, and K155A/G/V/L/S/W/Y.

In the present application, the DR5 domain variant may comprise amino acid mutations selected from any one of the following groups: (1) L114A and R115A (SEQ ID NO: 1); (2) R154A and K155A (SEQ ID NO: 2); (3) I87L, D90S, T105N, D109H, and D148N (SEQ ID NO: 3); (4) I87A, D90S, T105N, D109S, L114V, and E147S (SEQ ID NO: 4); (5) I87A, E89N, D90N, D109Q, and E147Y (SEQ ID NO: 5); (6) I87A, E89Q, D90S, T105S, D109S, L114V, and D148K (SEQ ID NO: 6); (7) D90S, T105N, D109E, L114I, D148E, and R154Y (SEQ ID NO: 7); (8) D90S, D109N, and D148E (SEQ ID NO: 8); (9) I87V, D90N, and R154Y (SEQ ID NO: 9); (10) T105S, N108Q, and F112Y (SEQ ID NO: 10); (11) L114V, E147Q, and E151S (SEQ ID NO: 11); (12) L114G and R115G (SEQ ID NO: 12); (13) L114V and R115V (SEQ ID NO: 13); (14) R115L (SEQ ID NO: 14); (15) L114S and R115S (SEQ ID NO: 15); (16) L114W and R115W (SEQ ID NO: 16); (17) L114Y and R115Y (SEQ ID NO: 17); (18) R154G and K155G (SEQ ID NO: 18); (19) R154V and K155V (SEQ ID NO: 19); (20) R154L and K155L (SEQ ID NO: 20); (21) R154S and K155S (SEQ ID NO: 21); (22) R154W and K155W (SEQ ID NO: 22); and (23) R154Y and K155Y (SEQ ID NO: 23).

In the present application, the DR5 domain variant may comprise an amino acid sequence as set forth in any one of SEQ ID NOs: 1-23, or an amino acid sequence having at least 80% (e.g., at least 85%, at least 90%, at least 91%, at least 92%, at least 93%, at least 94%, at least 95%, at least 96%, at least 97%, at least 98%, at least 99%, or at least 100%) sequence homology to the amino acid sequences as set forth in any one of SEQ ID NOs: 1-23.

In another aspect, the present application further provides a fusion protein comprising the DR5 domain variant described in the present application.

In the present application, the fusion protein may further comprise other functional proteins.

For example, the functional protein may comprise an immunoglobulin Fc region. For example, the fusion protein may comprise a DR5 domain and an immunoglobulin Fc region. For example, the immunoglobulin Fc region may comprise the Fc region of IgG. For example, the IgG may be selected from IgG1, IgG2, IgG3, and IgG4. For example, the IgG can be IgG1. For example, the IgG can be IgG4.

In the present application, the immunoglobulin Fc region may further comprise one or more amino acid mutations. For example, the amino acid mutation can be any amino acid mutation known to those skilled in the art. For example, the position 265 in the Fc region of IgG1 can comprise an amino acid mutation (SEQ ID NO: 99). For example, the Fc region of IgG4 comprises the amino acid mutation S228P.

For example, the immunoglobulin Fc region of the fusion protein may be directly linked to the DR5 domain variant. In the present application, the immunoglobulin Fc region of the fusion protein may be indirectly linked to the DR5 domain variant. For example, the N-terminus of the immunoglobulin Fc region of the fusion protein may be directly or indirectly linked to the C-terminus of the DR5 domain variant. For example, the C-terminus of the immunoglobulin Fc region of the fusion protein may be directly or indirectly linked to the N-terminus of the DR5 domain variant. For example, the indirect linking can be a case of linking via a linker. For example, the linker can comprise peptide linker. For example, the fusion protein may comprise the amino acid sequence as set forth in any one of SEQ ID NOs: 24-92.

For example, the functional protein may comprise a protein capable of extending the half-life of a drug in vivo. The protein capable of extending the half-life of a drug in vivo may be directly linked to the DR5 domain. The protein capable of extending the half-life of a drug in vivo can also be indirectly linked the DR5 domain.

For example, the functional protein may comprise human serum albumin. For example, the fusion protein may comprise a DR5 domain variant and human serum albumin. For example, the human serum albumin of the fusion protein may be directly linked to the DR5 domain variant. In the present application, the human serum albumin of the fusion protein may be indirectly linked to the DR5 domain variant. For example, the N-terminus of the human serum albumin of the fusion protein may be directly or indirectly linked to the C-terminus of the DR5 domain variant. For example, the C-terminus of the human serum albumin of the fusion protein may be directly or indirectly linked to the N-terminus of the DR5 domain variant. For example, the indirect linking can be a case of linking via a linker. For example, the linker can comprise peptide linker.

## Nucleic Acid Molecules, Vectors, Cells, Pharmaceutical Compositions, Preparation Methods and Applications

In another aspect, the present application further provides one or more isolated nucleic acid molecules which may encode the DR5 domain variant or the fusion protein described in the present application. In another aspect, the present application provides one or more vectors, which may comprise one or more nucleic acid molecules described in the present application. In another aspect, the present application provides a cell (e.g., a host cell) that may comprise the nucleic acid molecule described in the present application or the vector described in the present application.

The nucleic acid molecule according to the present application can be isolated. For example, it can be produced or synthesized by: (i) amplification in vitro, such as amplification by polymerase chain reaction (PCR), (ii) clonal recombination, (iii) purification, such as by enzymatic digestion and gel electrophoresis fractionation, or (iv) synthesis, such as chemical synthesis. In some embodiments, the isolated nucleic acid is a nucleic acid molecule prepared by recombinant DNA technology. In the present application, nucleic acids encoding the antibodies or antigen-binding fragments thereof can be prepared by a variety of methods known in the art, including but not limited to, overlap extension PCR using restricted fragment manipulation or using synthetic oligonucleotides. For specific operations, see Sambrook et al., Molecular Cloning, A Laboratory Manual, Cold Spring Harbor Laboratory Press, Cold Spring Harbor, N.Y., 1989; and Ausube et al., Current Protocols in Molecular Biology, Greene Publishing and Wiley-Interscience, New York N.Y., 1993.

In the present application, the vector may include a vector primarily used for insertion of DNA or RNA into cells, a vector primarily used for replication of DNA or RNA, and a vector primarily used for expression, transcription and/or translation of DNA or RNA. The vectors also include vectors having a variety of the above-mentioned functions. The vectors may be polynucleotides capable of being transcribed and translated into a polypeptide when introduced into suitable host cells. Generally, the vectors can produce the desired expression products by culturing appropriate host cells comprising the vectors. In the present application, one or more of the nucleic acid molecules may be comprised in the vectors. In addition, the vectors may further comprise other genes, such as marker genes which allow the vectors to be selected in suitable host cells and under suitable conditions. In addition, the vectors may further comprise expression control elements that allow correct expression of coding regions in an appropriate host. Such control elements are well known to those skilled in the art, and may include, for example, a promoter, a ribosome binding site, an enhancer, and other control elements that regulate gene transcription or mRNA translation. In some embodiments, the expression control sequences are regulatable elements. The specific structures of the expression control sequences may vary depending on species or the function of cell types, but generally include a 5' non-transcribed sequence and 5' and 3' non-translated sequences involved in the initiation of transcription and translation, respectively, for example, a TATA box, a caped sequence, and a CAAT sequence. For example, the 5' non-transcribed expression control sequence may comprise a promoter region, and the promoter region may comprise a promoter sequence for transcriptionally controlling the functionally linked nucleic acid.

In the present application, the host cells can include progeny of a single host cell. Due to natural, accidental or intentional mutations, the progeny cells may not necessarily be completely identical in morphology or genome to the original parent cells, but they can express the antibodies or antigen-binding fragments thereof described in the present application. The host cells can be obtained by transfecting cells in vitro using the vectors described in the present application. The host cells can be prokaryotic cells (such as E. coli) or eukaryotic cells (such as yeast cells, such as COS cells, Chinese hamster ovary (CHO) cells, HeLa cells, HEK293 cells, COS-1 cells, NS0 cells or myeloma cells). The vectors described in the present application may be introduced into the host cells by methods known in the art, for example, electroporation, lipofectine transfection, lipofectamin transfection, etc.

In another aspect, the present application further provides a method for preparing the DR5 domain variant or the fusion protein, which the method may comprise culturing the cells under conditions that allow the expression of the fusion protein. For example, appropriate culture medium, appropriate temperature, and culture time, etc. can be used, and these methods are understood by those of ordinary skill in the art.

In another aspect, the present application further provides a pharmaceutical composition which may comprise the DR5 domain variant, the fusion protein, the nucleic acid molecule, the vector and/or the cell, and optionally a pharmaceutically acceptable carrier.

For example, the pharmaceutically acceptable carrier may include buffers, antioxidants, preservatives, polypeptides with low molecular weight, proteins, hydrophilic polymers, amino acids, sugars, chelating agents, counter-ions, metal complexes, and/or nonionic surfactants, etc.

In the present application, the pharmaceutical composition can be formulated with a pharmaceutically acceptable carrier or diluent and any other known adjuvants and excipients according to conventional techniques in the art, for example, operating according to the techniques disclosed in Remington: The Science and Practice of Pharmacy, 19th Edition, Edited by Gennaro, Mack Publishing Co., Easton, PA, 1995.

In some embodiments, the pharmaceutical composition may further contain more than one active compounds, which are typically those active compounds that have complementary activity and do not adversely affect each other.

In some embodiments, the pharmaceutical composition can be administered parenterally, percutaneously, intracavitary, intravenously, intrathecally, and/or intranasally, or injected directly into a tissue. For example, the pharmaceutical composition can be administered to a patient or subject by infusion or injection. In some embodiments, the administration of the pharmaceutical composition can be performed in various ways, such as intravenous, intraperitoneal, subcutaneous, intramuscular, topical, or intradermal administration. In some embodiments, the pharmaceutical composition can be administered uninterruptedly. The uninterrupted (or continuous) administration can be achieved by a small pump system worn by a patient to measure the therapeutic agent flowing into the patient's body, as described in WO 2015/036583.

In another aspect, the present application further provides a method for preventing and/or treating diseases and/or disorders, comprising administering the DR5 domain variant, the fusion protein, the nucleic acid molecule, the vector, the cell, and/or the pharmaceutical composition to a subject in need thereof.

In the present application, the diseases and/or disorders may include the diseases and/or disorders associated with the TRAIL/DR5 pathway-induced apoptosis.

In the present application, the diseases and/or disorders may include myocardial infarction and/or myocardial infarction-reperfusion injury. In the present application, the diseases and/or disorders can include cerebral infarction.

In another aspect, the present application further provides use of the DR5 domain variant, the fusion protein, the nucleic acid molecule, the vector, the cell, and/or the pharmaceutical composition in preparation of a medicament which is used for preventing and/or treating diseases and/or disorders.

In the present application, the diseases and/or disorders may include the diseases and/or disorders associated with the TRAIL/DR5 pathway-induced apoptosis. In the present application, the diseases and/or disorders can include cerebral infarction.

In the present application, the diseases and/or disorders may include myocardial infarction and/or myocardial infarction-reperfusion injury.

In another aspect, the present application further provides the DR5 domain variant, the fusion protein, the nucleic acid molecule, the vector, the cell, and/or the pharmaceutical composition, for use in preventing and/or treating diseases and/or disorders.

In the present application, the diseases and/or disorders may include the diseases and/or disorders associated with the TRAIL/DR5 pathway-induced apoptosis.

In the present application, the diseases and/or disorders may include myocardial infarction and/or myocardial infarction-reperfusion injury. In the present application, the diseases and/or disorders can include cerebral infarction.

In another aspect, the present application further provides a method for blocking and/or closing the TRAIL/DR5 pathway, comprising administering the fusion protein or the pharmaceutical composition.

In some instances, the method can be an in vivo method.

In some instances, the method can be an in vitro method.

In some instances, the method can be an ex vivo method.

In some instances, the method can be a method not aimed at the diagnosis or treatment of diseases.

Without intending to be limited by any theory, the following examples are only intended to explain the variant, the fusion protein, the preparation method, the use, and the like of the present application, and are not intended to limit the scope of the invention of the present application.

**EXAMPLES**

**Example 1 Screening for DR5 Variants**

The truncated domain (SEQ ID NO: 94) of human DR5 variant 1 (NP_003833.4) was obtained, and the structure of the truncated domain interacting with TRAIL (NP_003801.1) was constructed using software. The sites involved in the interaction and the interaction pattern of the two proteins were theoretically analyzed, and the amino acid sites in the truncated domain that directly or indirectly involve in the interaction with TRAIL were determined to be I87, E89, D90, T105, N108, D109, L114, R115, E147, D148, E151, R154, and K155. The positions of these amino acid residues were numbered based on the amino acid sequence as set forth in SEQ ID NO: 96. Random mutation was made on these action sites and a mutant library was constructed. This mutant library was then cloned into the vector pTM. The pTM vector contains a signal peptide and a transmembrane region sequence, which can display the gene cloned into the vector on the cell surface.

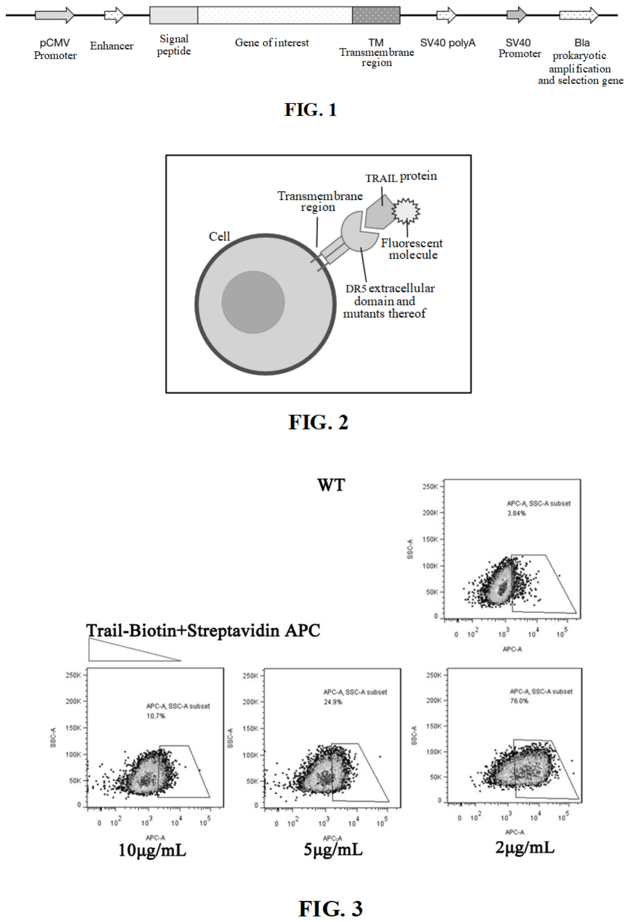

The constructed mutant library expression vector was transfected into CHO cells (ATCC), so that the mutant library was displayed and expressed on the cell surface. Then, TRAIL protein was fluorescently labeled with APC to obtain APC-TRAIL. Based on the binding activity between the APC-TRAIL and mutants of the truncated domain on the surface of CHO cells, the mutants that could bind to the APC-TRAIL were enriched and screened by using flow cytometry. The specific principle of screening can be seen in Fig. 2, in which the truncated domain and the mutants bind to the TRAIL protein bearing the fluorescent molecule, and the binding results can be reflected by the level of the fluorescent molecule.

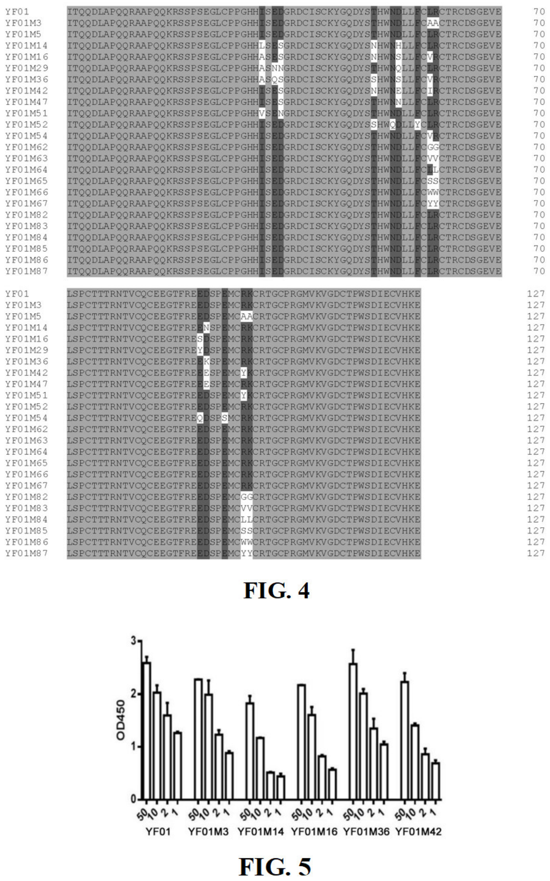

After three rounds of screening and enrichment, cells with strong binding to the APC-TRAIL were collected (as shown in Fig. 3). Then their mRNAs were extracted, cDNAs were obtained after reverse transcription, and the genes of the truncated domain mutants were sequenced and analyzed (as shown in Fig. 4). The sequencing results show that different mutation combinations existed at the aforementioned sites I87, E89, D90, T105, N108, D109, L114, R115, E147, D148, E151, R154, and K155.

From the results it can be seen that by introducing different mutation combinations at residues I87, E89, D90, T105, N108, D109, L114, R115, E147, D148, E151, R154, and K155, novel truncated domain mutants that may specifically recognize the DR5 can be obtained.

According to further analysis of the mutation sites, it can revealed that the amino acid residues mutated at each site were: I87A/V/L, E89N/Q, D90N/S, T105N/S, N108Q, D109H/S/Q/E, F112Y, L114A/G/V/S/W/Y, R115A/G/V/L/S/W/Y, E147S/Y, D148N/K/E, E151S, R154A/G/V/L/S/W/Y, and K155A/G/V/L/S/W/Y.

The amino acid sequence as set forth in SEQ ID NO: 94 was mutated and the DR5 domain variants were named as M3 (SEQ ID NO: 1), M5 (SEQ ID NO: 2), M14 (SEQ ID NO: 3), M16 (SEQ ID NO: 4), M29 (SEQ ID NO: 5), M36 (SEQ ID NO: 6), M42 (SEQ ID NO: 7), M47 (SEQ ID NO: 8), M51 (SEQ ID NO: 9), M52 (SEQ ID NO: 10), M54 (SEQ ID NO: 11), M62 (SEQ ID NO: 12), M63 (SEQ ID NO: 13), M64 (SEQ ID NO: 14), M65 (SEQ ID NO: 15), M66 (SEQ ID NO: 16), M67 (SEQ ID NO: 17), M82 (SEQ ID NO: 18), M83 (SEQ ID NO: 19), M84 (SEQ ID NO: 20), M85 (SEQ ID NO: 21), M86 (SEQ ID NO: 22), and M87 (SEQ ID NO: 23).

**Example 2 Binding Activity Assay (ELISA) of Fusion Proteins**

The ectodomain of human DR5 variant 1 in Example 1 (or referred to as wild-type DR5 truncated domain) and the variants of the DR5 ectodomain obtained in Example 1 were fused with human IgG1-Fc (whose amino acid sequence is as set forth in SEQ ID NO: 97) for expression, to obtain the corresponding DR5 ectodomain-human Fc fusion proteins (called shortly as fusion proteins). These fusion proteins were named as YF01M3 (SEQ ID NO: 24), YF01M5 (SEQ ID NO: 25), YF01M14 (SEQ ID NO: 26), YF01M16 (SEQ ID NO: 27), YF01M29 (SEQ ID NO: 28), YF01M36 (SEQ ID NO: 29), YF01M42 (SEQ ID NO: 30), YF01M47 (SEQ ID NO: 31), YF01M51 (SEQ ID NO: 32), YF01M52 (SEQ ID NO: 33), YF01M54 (SEQ ID NO: 34), YF01M62 (SEQ ID NO: 35), YF01M63 (SEQ ID NO: 36), YF01M64 (SEQ ID NO: 37), YF01M65 (SEQ ID NO: 38), YF01M66 (SEQ ID NO: 39), YF01M67 (SEQ ID NO: 40), YF01M82 (SEQ ID NO: 41), YF01M83 (SEQ ID NO: 42), YF01M84 (SEQ ID NO: 43), YF01M85 (SEQ ID NO: 44), YF01M86 (SEQ ID NO: 45), and YF01M87 (SEQ ID NO: 46), respectively.

As an example, YF01M3, YF01M14, YF01M16, YF01M36, YF01M42, and YF01M47 were selected for biological activity analysis. ELISA plates were coated with 10 g/ml target antigen TRAIL-His at 4°C overnight. After washing with PBST, 10% fetal bovine serum was added and blocked at 37°C for 1 hour. Then, YF01M3, YF01M14, YF01M16, YF01M36, YF01M42, and YF01M47 (with a concentration gradient of 1 μg/ml, 2 μg/ml, 10 μg/ml, and 50 μg/ml) were added thereto respectively and reaction was performed at 37°C for 1 hour. Then, the plates were washed with PBST, and horseradish peroxidase-labeled goat anti-human IgG secondary antibody (goat anti-human IgG HRP, Thermo Fisher Scientific) was added, and reaction was performed at room temperature for 30 minutes. Then, the plates were washed 5 times with PBST and any remaining liquid was patted dry with absorbent paper. 100 ml of TMB (eBioscience) was added to each well and placed in the dark at room temperature (20±5°C) for 1-5 minutes; 100 mL of 2 N $H_2SO_4$ was added to each well to terminate the reaction of substrates. The OD value was read at 450 nm using a plate reader, and the affinity of each fusion protein to the TRAIL molecule was analyzed (as shown in Fig. 5).

The results in Fig. 5 show that YF01M3, YF01M14, YF01M16, YF01M36, YF01M42, and YF01M47 could all bind to TRAIL with a certain binding affinity, and the effect is comparable to that of WT.

**Example 3 Affinity Analysis**

As an example, the affinity of each fusion protein such as YF01M3, YF01M5, and the like to the TRAIL molecule was determined using the Biacore method. The results are shown in Table 1 below.

Table 1. Biacore detection of the affinity to human TRAIL

| Ligand | hTRAIL | | |
|---|---|---|---|
| | Association rate $k_{on}$ (1/Ms) | Dissociation rate $k_{off}$ (1/s) | Affinity $K_D$ (M) |
| WT | 8.096E+05 | 4.272E-05 | 5.276E-11 |
| YF01M3 | 4.643+05 | 8.355E-05 | 1.799E-10 |
| YF01M5 | 3.987+05 | 1.311E-04 | 3.288E-10 |

The results in Table 1 show that each fusion protein such as YF01M3, YF01M5, and the like can all recognize the TRAIL molecule with a relatively high affinity.

**Example 4 Specifically Blocking the TRAIL-Induced Cell Killing by Fusion Proteins**

TRAIL can induce cell apoptosis in vitro by binding to the DR5 on the surface of Jurkat cells. We used flow cytometry to detect the blocking of this process by fusion proteins.

As an example, YF01M63, YF01M65, YF01M14, YF01M16, YF01M29, YF01M36, YF01M42, and YF01M47 were selected for analyzing the activity of blocking TRAIL killing, and YF01 protein was used as a control.

Jurkat cells in the logarithmic growth phase were collected and inoculated into a 24-well cell culture plate at $5×10^5$ cells/well. 200 ng/ml TRAIL (Novoprotein Scientific Inc.) and 0.25 mg/ml fusion protein were added to the 0.5 ml culture system. After evenly shaking, the cells were continuously cultured in the incubator for 6 h.

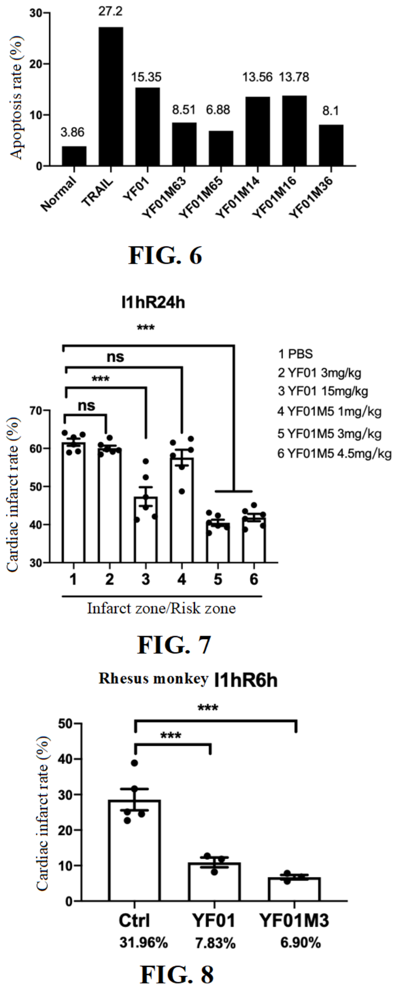

After staining with Annexin V-FITC and 7AAD apoptosis detection kit (Beyotime Biotechnology), the percents of total apoptotic cells of Annexin V$^+$7AAD$^-$ and Annexin V$^+$7AAD$^+$ were determined by flow cytometer (BD FACS Calibur) (the results are shown in Fig. 6).

The results in Fig. 6 indicate that at a concentration of 0.25 ug/ml, YF01M63, YF01M65, YF01M14, YF01M16, and YF01M36 could all effectively block the apoptosis induced by TRAIL at 200 ng/ml. Their inhibition effects on apoptosis were better than that of YF01 protein.

**Example 5 Detection of the Effects of Fusion Proteins on Protecting Against Myocardial Ischemia-Reperfusion Injury**

YF01M5 was used as an example to detect the protective effects of the fusion proteins against cardiac ischemia-reperfusion injury in rats in vivo.

Clean grade male Wistar rats (200-250 g body weight) were anesthetized by intraperitoneally injection of 10% chloral hydrate. Endotracheal intubation was performed. A ventilator was connected before opening the intercostal space. Parameters were adjusted with an inspiratory/expiratory ratio of 1:2, a respiratory rate of 90-120 times/minutes, and a tidal volume of 2-4 ml. A transverse incision was made at the 3rd-4th intercostal space on the left side of the sternum. The pericardium was incised to expose the heart. The left coronary vein at the junction of the pulmonary artery cone and the left auricular appendage was used as a landmark, and a needle was inserted at 0.1 cm from the midpoint of the lower edge of the left auricular appendage and ligation was performed. The ligature was loosened after a specific time. The color of myocardium turning red served as a sign of successful reperfusion. After surgery, 400,000 U penicillin per animal was routinely injected. The fusion proteins (calibration concentration) was administered 10 minutes before reperfusion, and the left ventricular myocardial tissue was sliced 24 h later. The range of myocardial infarction in each treatment group was observed by Evans blue/TTC staining, and pictures were taken with a digital camera. The area of the flat infarct lesion was measured using Image-Pro Plus 6.0 software. Wherein the Evans blue-rejected area was risk zone, and the TTC-stained white area was infarct zone. Relative infarct rate was used to judge the degree of cardiac damage. The cardiac infarct rate was calculated by the following formula:

$$\text{Relative infarct rate} = \frac{\text{Area of infarct zone}}{\text{Area of risk zone}} \times 100\%$$

There were no less than 5 animals in each group. The results are shown in Fig. 7.

The results shown in Fig. 7 indicate that YF01M5 protein protected against cardiac ischemia-reperfusion injury in rats in a dose-dependent manner, and had a protection effect against myocardial infarction (26%) at a dose of 3 mg/kg comparable to that of YF01 (WT) protein at a dose of 15 mg/kg (24%), indicating that it has significant dose superiority.

YF01M3 was used as an example to detect the protective effects of the fusion proteins against cardiac ischemia-reperfusion injury in rhesus monkeys in vivo.

Animal anesthesia and preoperative preparation: the monkey was fixed and anesthetized by intramuscular injection of 0.15 ml/kg Lumianning (Huamu Company, drug had a concentration of 100 mg/ml, and diluted 10 times before use), and its number (on the ear) was recorded. The monkey was fixed on the operating table after weighing, and cardiac function was measured by color Doppler ultrasound. The intravenous channel was opened, physiological saline was dripped, and sufentanil, etomidate, and vecuronium bromide were injected intravenously. Endotracheal intubation was performed. Anticoagulated blood was drawn from the groin before the operation, and blood cells, cardiac function (CK-MB), kidney function (creatinine and urea nitrogen), and liver function (glutamic pyruvic transaminase and glutamic oxaloacetic transaminase) were detected by using a special animal hemacytometer and blood biochemical analyzer. The electrosurgical unit and electrodes were fixed, and the electrocardiogram, blood oxygen saturation, and blood pressure were recorded. The surgical site was disinfected with iodine complex.

Drug formulation: According to the weight of the rhesus monkey, the drugs at appropriate concentrations were formulated and randomly numbered. The injection dose was 2 mg/kg for YF01M3, and 6 mg/kg for YF01 (WT), and the injection volume was 5 mL. After formulation, the drugs were stored at 4°C in the dark.

Animal surgery: After the heart rate and other indicators of the rhesus monkey were stabilized, the skin and muscles were incised at the 1st-2nd intercostal space under the left breast (5th-6th intercostal space on the left side) to expose the heart. The pericardium was incised and suspended to expose the left anterior descending coronary artery. After the heart rate and other indicators were stabilized, the blood vessel was ligated at the 1/3 near the apex (below the oblique branch) of the left anterior descending coronary artery using sutures and a urinary catheter. The electrocardiogram, blood oxygen saturation, and blood pressure were recorded 5 minutes later. (5) Blood was collected to test blood cells, heart function, kidney function, and liver function after 50 minutes. 5 mL of drug was slowly injected intravenously (the drug number was recorded) for 2-3 minutes after 55 minutes. The ligature was loosened and the blood vessels were recanalized at 60 minutes; Blood was collected to test blood cells, heart function, kidney function, and liver function at 120 minutes, 240 minutes, and 360 minutes. Up to 6 hours after reperfusion, a large dose of anesthetic was administered, and air injection was performed to sacrifice the rhesus monkey.

Tissue acquisition and cardiac TTC staining: heart was obtained, perfused with physiological saline, and then frozen in a -20°C freezer overnight. A certain amount of TTC powder was weighed and dissolved in $1\times$ PBS (protected from light) to formulate a working solution with a concentration of 1%. The prepared TTC staining solution was placed in a 37°C incubator protected from light to preheat for 15-30 minutes. The monkey's heart tissue was removed from the -20°C freezer and waited for its surface to soften slightly before slicing and staining. 3-4 mm heart sections were sliced evenly from the apex toward the direction of the ligature. The heart sections were placed in the preheated TTC staining solution and incubated in a 37°C incubator protected from light for 15 minutes. 15 minutes later, the heart sections were turned over once to ensure uniform staining. (7) After staining was complete, the stained heart tissues were photographed.

Photographing and weighing after TTC staining: each heart section was photographed for each monkey. The right ventricle was removed and the weights of the heart sections were recorded. The white infarct zone in the sections were obtained and weighed. The cardiac damage was judged based on the relative infarct rate. The cardiac infarct rate was calculated by the following formula:

$$\text{Relative infarct rate } = \frac{Area\ of\ infarct\ zone}{Area\ of\ left\ ventricular} \times 100\%$$

There were no less than 3 animals in each group. The results are shown in Fig. 8.

The results in Fig. 8 indicate that YF01M3 protein at a dose of 2 mg/kg significantly protected against cardiac ischemia-reperfusion injury in rhesus monkeys (78%), which was comparable to that of YF01 (WT)protein at a dose of 6 mg/kg (75%), indicating that it has a dose superiority.

**Example 6 Effects of Fusion to Different Subtypes of IgG Fc on the Activity of Fusion Proteins**

According to the method of constructing the fusion proteins in Example 2, the DR5 domain mutants obtained in Example 1, i.e., M3, M5, M14, M16, M29, M36, M42, M47, M51, M52, M54, M62, M63, M64, M65, M66, M67, M82, M83, M84, M85, M86 and M87, were respectively fused to human IgG4-Fc (its amino acid sequence was as set forth in SEQ ID NO: 98) for expression, to obtain the corresponding DR5 mutant truncated domain-human Fc fusion proteins (called shortly as fusion proteins). These fusion proteins were named as YF01M3G4 (SEQ ID NO: 47), YF01M5G4 (SEQ ID NO: 48), YF01M14G4 (SEQ ID NO: 49), YF01M16G4 (SEQ ID NO: 50), YF01M29G4 (SEQ ID NO: 51), YF01M36G4 (SEQ ID NO: 52), YF01M42G4 (SEQ ID NO: 53), YF01M47G4 (SEQ ID NO: 54), YF01M51G4 (SEQ ID NO: 55), YF01M52G4 (SEQ ID NO: 56), YF01M54G4 (SEQ ID NO: 57), YF01M62G4 (SEQ ID NO: 58), YF01M63G4 (SEQ ID NO: 59), YF01M64G4 (SEQ ID NO: 60), YF01M65G4 (SEQ ID NO: 61), YF01M66G4 (SEQ ID NO: 62), YF01M67G4 (SEQ ID NO: 63), YF01M82G4 (SEQ ID NO: 64), YF01M83G4 (SEQ ID NO: 65), YF01M84G4 (SEQ ID NO: 66), YF01M85G4 (SEQ ID NO: 67), YF01M86G4 (SEQ ID NO: 68), and YF01M87G4 (SEQ ID NO: 69), respectively.

The DR5 domain mutants obtained in Example 1, i.e., M3, M5, M14, M16, M29, M36, M42, M47, M62, M63, M64, M65, M66, M67, M82, M83, M84, M85, M86 and M87, were respectively fused to the mutated human IgG1-Fc (its amino acid sequence was as set forth in SEQ ID NO: 99) for expression, to obtain the corresponding DR5 mutant truncated domain-human Fc fusion proteins (called shortly as fusion proteins). These fusion proteins were named as YF01M3G1m (SEQ ID NO: 70), YF01M5G1m (SEQ ID NO: 71), YF01M14G1m (SEQ ID NO: 72), YF01M16G1m (SEQ ID NO: 73), YF01M29G1m (SEQ ID NO: 74), YF01M36G1m (SEQ ID NO: 75), YF01M42G1m (SEQ ID NO: 76), YF01M47G1m (SEQ ID NO: 77), YF01M51G1m (SEQ ID NO: 78), YF01M52G1m (SEQ ID NO: 79), YF01M54G1m (SEQ ID NO: 80), YF01M62G1m (SEQ ID NO: 81), YF01M63G1m (SEQ ID NO: 82), YF01M64G1m (SEQ ID NO: 83), YF01M65G1m (SEQ ID NO: 84), YF01M66G1m (SEQ ID NO: 85), YF01M67G1m (SEQ ID NO: 86), YF01M82G1m (SEQ ID NO: 87), YF01M83G1m (SEQ ID NO: 88), YF01M84G1m (SEQ ID NO: 89), YF01M85G1m (SEQ ID NO: 90), YF01M86G1m (SEQ ID NO: 91), and YF01M87G1m (SEQ ID NO: 92), respectively.

As an example, YF01M3G4 and YF01M3G1m were selected for biological activity analysis.

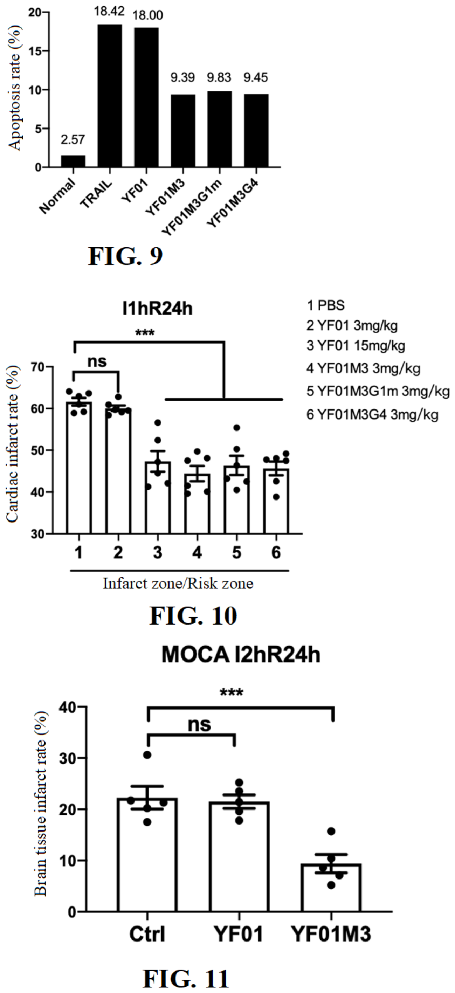

The activity of YF01M3G4 and YF01M3G1m in blocking TRAIL-induced Jurkat cell apoptosis was analyzed according to the method for specifically blocking TRAIL-induced cell killing in Example 4. The results are shown in Fig. 9. The results in Fig. 9 indicate that both YF01M3G4 and YF01M3G1m at a dose of 0.125 mg/ml can block the apoptosis induced by 200 ng/ml TRAIL, and which effects are comparable to that of M3-1 and superior to that of YF01 (WT).

The effects of YF01M3G4 and YF01M3G1m on protecting against myocardial ischemia-reperfusion injury in vivo were analyzed according to the method for protecting against myocardial ischemia-reperfusion injury in rats in Example 5. The results are shown in Fig.10. The results in Fig. 10 indicate that both YF01M3G4 and YF01M3G1m can protect against myocardial ischemia-reperfusion injury in rats at a dose of 3 mg/kg, and which effects are comparable to that of 3 mg/kg M3 and 15 mg/kg WT.

From the above results, it can be seen that the fusion to different subtypes of IgG Fc has no significant impact on the activity of the fusion proteins constructed in the present application.

**Example 7 The Effects of Fusion Proteins** on **Protecting Against Cerebral Infarction/Reperfusion Injury**

Taking YF01M3 as an example, the protective effects against brain injury caused by middle artery occlusion and reperfusion were tested.

The rats were placed in a clean-grade environment with an ambient temperature of $24 \pm 2°C$ and a humidity of $45 \pm 5\%$, with a 12-h day and night alternation, and were fed with a standard diet. The rats were randomly divided into a PBS control group and a M3-1 treatment group, with no less than 5 rats in each group. The rats were fasted for 12 hours before the experiment, but were allowed to drink water freely. After the rats were anesthetized with pentobarbitone sodium, they were fixed in a supine position, and their hair were roughly removed for skin preparation (depilatory cream could also be used to remove hair). The neck skin was moistened and disinfected with iodophor. An opening was made along the right side of the middle part below the lower jaw bone of the neck, and each layer of tissues were bluntly separated. The membrane wrapped around the vagus nerve and common carotid artery was scissored with an ophthalmic scissor. The vagus nerve close to the common carotid artery was bluntly separated with a glass needle to expose the right common carotid artery (CCA). The external carotid artery (ECA) and internal carotid artery (ICA) were separated at the distal end of the common carotid artery. A 4-0 thread was passed through the proximal end of the common carotid artery (CCA) and below the internal carotid artery (ICA) to control the blood flow in the artery during thread plug insertion (an artery clamp could also be used to stop bleeding). Two 5-0 threads were passed blow the external carotid artery (ECA), and ligation was performed at the distal end twice. After ligation, the blood vessel was cut from the middle. The cotton threads of the internal carotid artery (ICA) and common carotid artery (CCA) were slightly tightened to prevent bleeding during the thread plug insertion. The direction of the ECA stump was adjusted to be in a straight line with the ICA, and an incision was made at the bifurcation of the ECA near the CCA to insert a thread plug toward the intracranial direction. The thread plug was made of 4-0 nylon thread with a diameter of 28 m and a certain hardness. The front segment inserted into the blood vessel was made into a slightly bulged spherical shape. The plug insertion was stopped when resistance was felt at an insertion depth of about $18.5 \pm 0.5$ mm. Ligation was performed below the thread plug incision to prevent bleeding from the blood vessel incision. The hemostatic forceps was removed, the separated tissues were restored to its original state, and a heating pad was placed to keep warm after suturing After surgery, the rats were irradiated with a warm lamp or kept warm with a heating pad to maintain their rectal temperature at around 37°C.

After 30 minutes of plug insertion, the ischemic side of the brain was injected intracranially using a brain stereotaxic apparatus. The injection dose was 10 μl and the injection weight of M3-1 was 60 μg. The brain was sutured and disinfected. After 2 hours of plug insertion, the thread plug was pulled out while the rat was anesthetized again, and the thin thread below the external carotid artery was tied tightly. An appropriate amount of antibiotics was sprinkled on the skin of incision and sutured in a standard manner. The rats were put back into the cages, kept warm, and fed with 5% aqueous glucose solution.

Infarct volume was quantified by 2,3,5-triphenyltetrazolium chloride (TTC) staining. At 24 hours after MCAO surgery, the rats were deeply anesthetized and the brain tissue was removed. The brain tissue was evenly cut into 6 slices of coronal sections with 2 mm thickness along the coronal plane using a brain slicing mold. The sections were gently pushed into the pre-prepared 1% TTC staining solution (in a 6-well plate for cell culture) by using a tweezer. It should make sure that the sections were attached to the bottom of the 6-well plate in the same coronal plane direction. The staining solution should cover the brain tissue sections. The 6-well plate was covered with a light shield and placed in a 37°C water bath for staining for 20 minutes. The sections were turned over with a tweezer and continuously stained for 5-10 minutes. The sections were then taken out and fixed in 4% paraformaldehyde overnight. The TTC-stained brain sections were placed in a uniform coronal plane from the cerebellum to the olfactory bulb in order, and photographed using a digital camera. The area of the flat infarct lesion was measured by using Image-Pro Plus 6.0 software, and the relative infarct volume was calculated by the following formula (the results are shown in Fig. 11):

$$Relative\ infarct\ volume$$

$$= \frac{2\,mm \times area\ of\ contralateral\ hemisphere\ zone - 2\,mm \times area\ of\ ipsilateral\ hemisphere\ noninfarct\ zone}{2\,mm \times area\ of\ contralateral\ hemisphere\ zone} \times 100\%$$

The results in Fig. 11 indicate that YF01M3 can significantly protect against the tissue damage caused by cerebral infarction/reperfusion.

**Example 8 The Effects of Fusion Proteins Via Intramuscular Injection on Protecting Against Myocardial Infarction/Reperfusion Injury**

YF01M5 was used as an example to detect the protective effects of the fusion proteins by intramuscular administration against cardiac ischemia-reperfusion injury in rats.

The modeling and evaluation of the cardiac ischemia-reperfusion injury in rats were the same as described in Example 5. The calibration drug was administered by intramuscular injection into the hind limb 10 minutes before reperfusion, and the drug volume was 200 ul.

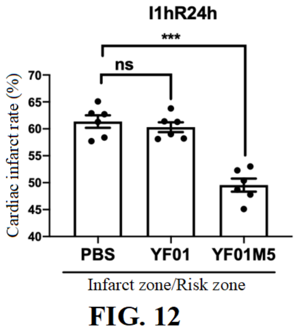

The results in Fig. 12 indicate that, by intramuscular injection, YF01M5 can significantly protect against the myocardium tissue damage caused by cerebral infarction/reperfusion.

**Claims**

1.   A death receptor 5 (DR5) domain variant having one or more of the following properties:

     (1) being capable of binding to TRAIL;
     (2) blocking TRAIL/DR5 pathway-induced apoptosis;
     (3) reducing the area of myocardial infarction in cardiac ischemia and/or ischemia-reperfusion; and
     (4) having protective effects against cerebral infarction.

2.   The DR5 domain variant of claim 1, wherein said DR5 domain comprises an extracellular domain of DR5.

3.   The DR5 domain variant of claim 1 or 2, wherein said DR5 domain variant comprises an amino acid mutation(s) at one or more amino acid sites from positions 86 to 155 compared to an extracellular domain of human DR5 variant 1 (SEQ ID NO: 94).

4.   The DR5 domain variant of any one of claims 1-3, wherein said death receptor 5 (DR5) includes soluble death receptor 5 (sDR5).

5.   The DR5 domain variant of any one of claims 1-4, wherein said DR5 is human DR5.

6.   The DR5 domain variant of any one of claims 3-5, wherein said extracellular domain of the human DR5 variant 1 comprises the amino acid sequence as set forth in SEQ ID NO: 94.

7.   The DR5 domain variant of any one of claims 1-6, comprising an amino acid mutation(s) at one or more amino acid sites from positions 86 to 92.

8.   The DR5 domain variant of any one of claims 1-7, comprising an amino acid mutation(s) at one or more amino acid sites from positions 105 to 112.

9.   The DR5 domain variant of any one of claims 1-8, comprising an amino acid mutation(s) at one or more amino acid sites from positions 114 to 155.

10.  The DR5 domain variant of any one of claims 1-9, comprising an amino acid mutation(s) at one or both amino acid sites from positions 114 to 115.

11.  The DR5 domain variant of any one of claims 1-10, comprising an amino acid mutation(s) at one or more amino acid sites from positions 147 to 152.

12.  The DR5 domain variant of any one of claims 1-11, comprising an amino acid mutation(s) at one or both amino acid sites from positions 154 to 155.

13.  The DR5 domain variant of any one of claims 1-12, comprising an amino acid mutation(s) at one or more amino acid sites selected from the group consisting of I87, E89, D90, T105, N108, D109, L114, R115, E147, D148, E151, R154, and K155, compared to the amino acid sequence as set forth in SEQ ID NO: 94.

14.  The DR5 domain variant of any one of claims 1-13, comprising a mutation(s) at the amino acid site(s) selected from any one of the following groups, compared to the amino acid sequence as set forth in SEQ ID NO: 94:

(1) L114 and R115;
(2) R154 and K155;
(3) I87, D90, T105, D109, and D148;
(4) I87, D90, T105, D109, L114, E147;
(5) I87, E89, D90, D109, E147;
(6) I87, E89, D90, T105, D109, L114, D148;
(7) D90, T105, D109, L114, D148, R154;
(8) D90, D109, and D148;
(9) I87, D90, and R154;
(10) T105, N108, and F112;
(11) L114, E147, and E151; and
(12) R115.

15. The DR5 domain variant of any one of claims 1-14, wherein in said amino acid mutation(s), the mutated amino acid is a polar amino acid.

16. The DR5 domain variant of any one of claims 1-14, wherein in said amino acid mutation(s), the mutated amino acid is a neutral amino acid.

17. The DR5 domain variant of any one of claims 1-14, wherein in said amino acid mutation(s), the mutated amino acid is an acidic amino acid.

18. The DR5 domain variant of any one of claims 1-14, wherein in said amino acid mutation(s), the mutated amino acid is a basic amino acid.

19. The DR5 domain variant of any one of claims 1-14, wherein in said amino acid mutation(s), the mutated amino acid is a non-polar amino acid.

20. The DR5 domain variant of any one of claims 1-14, wherein in said amino acid mutation(s), the mutated amino acid is an amino acid with a relatively small side chain volume.

21. The DR5 domain variant of any one of claims 1-20, comprising one or more amino acid mutations selected from the group consisting of I87A/V/L, E89N/Q, D90N/S, T105N/S, N108Q, D109H/S/Q/E, F112Y, L114A/G/V/S/W/Y, R115A/G/V/L/S/W/Y, E147S/Y, D148N/K/E, E151S, R154A/G/V/L/S/W/Y, and K155A/G/V/L/S/W/Y, compared to the amino acid sequence as set forth in SEQ ID NO: 94.

22. The DR5 domain variant of any one of claims 1-21, comprising an amino acid mutation(s) selected from any one of the following groups, compared to the amino acid sequence as set forth in SEQ ID NO: 94:

(1) L114A and R115A;
(2) R154A and K155A;
(3) I87L, D90S, T105N, D109H, and D148N;
(4) I87A, D90S, T105N, D109S, L114V, and E147S;
(5) I87A, E89N, D90N, D109Q, and E147Y;
(6) I87A, E89Q, D90S, T105S, D109S, L114V, and D148K;
(7) D90S, T105N, D109E, L114I, D148E, and R154Y;
(8) D90S, D109N, and D148E;
(9) I87V, D90N, and R154Y;
(10) T105S, N108Q, and F112Y;
(11) L114V, E147Q, and E151S;
(12) L114G and R115G;
(13) L114V and R115V;
(14) R115L;
(15) L114S and R115S;
(16) L114W and R115W;
(17) L114Y and R115Y;

(18) R154G and K155G;
(19) R154V and K155V;
(20) R154L and K155L;
(21) R154S and K155S;
(22) R154W and K155W; and
(23) R154Y and K155Y.

23. The DR5 domain variant of any one of claims 1-22, comprising the amino acid sequence as set forth in any one of SEQ ID NOs: 1-23.

24. A fusion protein, comprising the DR5 domain variant of any one of claims 1-23.

25. The fusion protein of claim 24, further comprising an immunoglobulin Fc region.

26. The fusion protein of claim 25, wherein said immunoglobulin Fc region comprises the Fc region of IgG.

27. The fusion protein of claim 26, wherein said IgG is selected from the group consisting of IgG and IgG4.

28. The fusion protein of any one of claims 25-27, wherein said immunoglobulin Fc region comprises the amino acid sequence as set forth in any one of SEQ ID NO: 97, SEQ ID NO: 98, and SEQ ID NO: 99.

29. The fusion protein of any one of claims 25-28, wherein said immunoglobulin Fc region is directly or indirectly linked to said DR5 domain.

30. The fusion protein of any one of claims 25-29, wherein said DR5 domain variant is located at the N-terminus of said immunoglobulin Fc region.

31. The fusion protein of any one of claims 25-29, wherein said DR5 domain variant is located at the C-terminus of said immunoglobulin Fc region.

32. The fusion protein of any one of claims 24-31, further comprising a protein capable of extending half-life.

33. The fusion protein of claim 32, wherein said protein capable of extending half-life comprises human serum albumin.

34. The fusion protein of claim 32 or 33, wherein said protein capable of extending half-life is directly or indirectly linked to said DR5 domain variant.

35. The fusion protein of any one of claims 32-34, wherein said protein capable of extending half-life is located at the N-terminus of said DR5 domain variant.

36. The fusion protein of any one of claims 32-34, wherein said protein capable of extending half-life is located at the C-terminus of said DR5 domain variant.

37. The fusion protein of any one of claims 24-36, comprising the amino acid sequence as set forth in any one of SEQ ID NOs: 24-92.

38. One or more isolated nucleic acid molecules, encoding the DR5 domain variant of any one of claims 1-23 or the fusion protein of any one of claims 24-37.

39. A vector, comprising the nucleic acid molecule of claim 38.

40. A cell, comprising the nucleic acid molecule of claim 38 or the vector of claim 39.

41. A method for preparing the DR5 domain variant of any one of claims 1-23 or the fusion protein of any one of claims 24-37, comprising culturing the cell of claim 40 under conditions that allow the expression of said DR5 domain variant or said fusion protein.

42. A pharmaceutical composition, comprising the DR5 domain variant of any one of claims 1-23, the fusion protein of any one of claims 24-37, the nucleic acid molecule of claim 38, the vector of claim 39, and/or the cell of claim 40, and optionally a pharmaceutically acceptable carrier.

43. The pharmaceutical composition of claim 42, further comprising other active ingredients.

44. A method for preventing and/or treating a disease and/or disorder, comprising administering the DR5 domain variant of any one of claims 1-23, the fusion protein of any one of claims 24-37, the nucleic acid molecule of claim 38, the vector of claim 39, the cell of claim 40, and/or the pharmaceutical composition of claim 42 or 43 to a subject in need thereof.

45. The method of claim 44, wherein said disease and/or disorder includes myocardial infarction and/or myocardial infarction-reperfusion injury.

46. The method of claim 44 or 45, wherein said disease and/or disorder includes cerebral infarction.

47. Use of the DR5 domain variant of any one of claims 1-23, the fusion protein of any one of claims 24-37, the nucleic acid molecule of claim 38, the vector of claim 39, the cell of claim 40, and/or the pharmaceutical composition of claim 42 or 43 in preparation of a medicament which is used for the prevention and/or treatment of a disease and/or disorder.

48. The use of claim 47, wherein said disease and/or disorder includes myocardial infarction and/or myocardial infarction-reperfusion injury.

49. The use of claim 47 or 48, wherein said disease and/or disorder includes cerebral infarction.

50. The DR5 domain variant of any one of claims 1-23, the fusion protein of any one of claims 24-37, the nucleic acid molecule of claim 38, the vector of claim 39, the cell of claim 40, and/or the pharmaceutical composition of claim 42 or 43, for use in the prevention and/or treatment of a disease and/or disorder.

51. The DR5 domain variant, the fusion protein, the nucleic acid molecule, the vector, the cell, and/or the pharmaceutical composition of claim 50, wherein said disease and/or disorder includes myocardial infarction and/or myocardial infarction-reperfusion injury.

52. The fusion protein, the nucleic acid molecule, the vector, the cell, and/or the pharmaceutical composition of claim 50 or 51, wherein said disease and/or disorder includes cerebral infarction.

53. A method for blocking the TRAIL/DR5 pathway, comprising administering the DR5 domain variant of any one of claims 1-23, the fusion protein of any one of claims 24-37, or the pharmaceutical composition of claim 42 or 43.

**FIG. 1**

**FIG. 2**

**FIG. 3**

```
YF01      ITQQDLAPQQRAAPQQKRSSPSEGLCPPGHHISEDGRDCISCKYGQDYSTHWNDLLFCLRCTRCDSGEVE   70
YF01M3    ITQQDLAPQQRAAPQQKRSSPSEGLCPPGHHISEDGRDCISCKYGQDYSTHWNDLLFCAACTRCDSGEVE   70
YF01M5    ITQQDLAPQQRAAPQQKRSSPSEGLCPPGHHISEDGRDCISCKYGQDYSTHWNDLLFCLRCTRCDSGEVE   70
YF01M14   ITQQDLAPQQRAAPQQKRSSPSEGLCPPGHHLSESGRDCISCKYGQDYSNHWNHLLFCLRCTRCDSGEVE   70
YF01M16   ITQQDLAPQQRAAPQQKRSSPSEGLCPPGHHASESGRDCISCKYGQDYSNHWNSLLFCVRCTRCDSGEVE   70
YF01M29   ITQQDLAPQQRAAPQQKRSSPSEGLCPPGHHASNNGRDCISCKYGQDYSTHWNQLLFCLRCTRCDSGEVE   70
YF01M36   ITQQDLAPQQRAAPQQKRSSPSEGLCPPGHHASQSGRDCISCKYGQDYSSHWNSLLFCVRCTRCDSGEVE   70
YF01M42   ITQQDLAPQQRAAPQQKRSSPSEGLCPPGHHISESGRDCISCKYGQDYSNHWNELLFCIRCTRCDSGEVE   70
YF01M47   ITQQDLAPQQRAAPQQKRSSPSEGLCPPGHHLSESGRDCISCKYGQDYSTHWNNLLFCLRCTRCDSGEVE   70
YF01M51   ITQQDLAPQQRAAPQQKRSSPSEGLCPPGHHVSFNGRDCISCKYGQDYSTHWNDLLFCLRCTRCDSGEVE   70
YF01M52   ITQQDLAPQQRAAPQQKRSSPSEGLCPPGHHISEDGRDCISCKYGQDYSSHWQDLLYCLRCTRCDSGEVE   70
YF01M54   ITQQDLAPQQRAAPQQKRSSPSEGLCPPGHHISEDGRDCISCKYGQDYSTHWNDLLFCVRCTRCDSGEVE   70
YF01M62   ITQQDLAPQQRAAPQQKRSSPSEGLCPPGHHISEDGRDCISCKYGQDYSTHWNDLLFCGGCTRCDSGEVE   70
YF01M63   ITQQDLAPQQRAAPQQKRSSPSEGLCPPGHHISEDGRDCISCKYGQDYSTHWNDLLFCVVCTRCDSGEVE   70
YF01M64   ITQQDLAPQQRAAPQQKRSSPSEGLCPPGHHISEDGRDCISCKYGQDYSTHWNDLLFCILCTRCDSGEVE   70
YF01M65   ITQQDLAPQQRAAPQQKRSSPSEGLCPPGHHISEDGRDCISCKYGQDYSTHWNDLLFCSSCTRCDSGEVE   70
YF01M66   ITQQDLAPQQRAAPQQKRSSPSEGLCPPGHHISEDGRDCISCKYGQDYSTHWNDLLFCWWCTRCDSGEVE   70
YF01M67   ITQQDLAPQQRAAPQQKRSSPSEGLCPPGHHISEDGRDCISCKYGQDYSTHWNDLLFCYYCTRCDSGEVE   70
YF01M82   ITQQDLAPQQRAAPQQKRSSPSEGLCPPGHHISEDGRDCISCKYGQDYSTHWNDLLFCLRCTRCDSGEVE   70
YF01M83   ITQQDLAPQQRAAPQQKRSSPSEGLCPPGHHISEDGRDCISCKYGQDYSTHWNDLLFCLRCTRCDSGEVE   70
YF01M84   ITQQDLAPQQRAAPQQKRSSPSEGLCPPGHHISEDGRDCISCKYGQDYSTHWNDLLFCLRCTRCDSGEVE   70
YF01M85   ITQQDLAPQQRAAPQQKRSSPSEGLCPPGHHISEDGRDCISCKYGQDYSTHWNDLLFCLRCTRCDSGEVE   70
YF01M86   ITQQDLAPQQRAAPQQKRSSPSEGLCPPGHHISEDGRDCISCKYGQDYSTHWNDLLFCLRCTRCDSGEVE   70
YF01M87   ITQQDLAPQQRAAPQQKRSSPSEGLCPPGHHISEDGRDCISCKYGQDYSTHWNDLLFCLRCTRCDSGEVE   70


YF01      LSPCTTTRNTVCQCEEGTFREEDSPEMCRKCRTGCPRGMVKVGDCTPWSDIECVHKE  127
YF01M3    LSPCTTTRNTVCQCEEGTFREEDSPEMCRKCRTGCPRGMVKVGDCTPWSDIECVHKE  127
YF01M5    LSPCTTTRNTVCQCEEGTFREEDSPEMCAACRTGCPRGMVKVGDCTPWSDIECVHKE  127
YF01M14   LSPCTTTRNTVCQCEEGTFREENSPEMCRKCRTGCPRGMVKVGDCTPWSDIECVHKE  127
YF01M16   LSPCTTTRNTVCQCEEGTFRESDSPEMCRKCRTGCPRGMVKVGDCTPWSDIECVHKE  127
YF01M29   LSPCTTTRNTVCQCEEGTFREYDSPEMCRKCRTGCPRGMVKVGDCTPWSDIECVHKE  127
YF01M36   LSPCTTTRNTVCQCEEGTFREEKSPEMCRKCRTGCPRGMVKVGDCTPWSDIECVHKE  127
YF01M42   LSPCTTTRNTVCQCEEGTFREEESPEMCYKCRTGCPRGMVKVGDCTPWSDIECVHKE  127
YF01M47   LSPCTTTRNTVCQCEEGTFREFESPEMCRKCRTGCPRGMVKVGDCTPWSDIECVHKE  127
YF01M51   LSPCTTTRNTVCQCEEGTFREEDSPEMCYKCRTGCPRGMVKVGDCTPWSDIECVHKE  127
YF01M52   LSPCTTTRNTVCQCEEGTFREEDSPEMCRKCRTGCPRGMVKVGDCTPWSDIECVHKE  127
YF01M54   LSPCTTTRNTVCQCEEGTFREQDSPSMCRKCRTGCPRGMVKVGDCTPWSDIECVHKE  127
YF01M62   LSPCTTTRNTVCQCEEGTFREEDSPEMCRKCRTGCPRGMVKVGDCTPWSDIECVHKE  127
YF01M63   LSPCTTTRNTVCQCEEGTFREEDSPEMCRKCRTGCPRGMVKVGDCTPWSDIECVHKE  127
YF01M64   LSPCTTTRNTVCQCEEGTFREEDSPEMCRKCRTGCPRGMVKVGDCTPWSDIECVHKE  127
YF01M65   LSPCTTTRNTVCQCEEGTFREEDSPEMCRKCRTGCPRGMVKVGDCTPWSDIECVHKE  127
YF01M66   LSPCTTTRNTVCQCEEGTFREEDSPEMCRKCRTGCPRGMVKVGDCTPWSDIECVHKE  127
YF01M67   LSPCTTTRNTVCQCEEGTFREEDSPEMCRKCRTGCPRGMVKVGDCTPWSDIECVHKE  127
YF01M82   LSPCTTTRNTVCQCEEGTFREEDSPEMCGGCRTGCPRGMVKVGDCTPWSDIECVHKE  127
YF01M83   LSPCTTTRNTVCQCEEGTFREEDSPEMCVVCRTGCPRGMVKVGDCTPWSDIECVHKE  127
YF01M84   LSPCTTTRNTVCQCEEGTFREEDSPEMCLLCRTGCPRGMVKVGDCTPWSDIECVHKE  127
YF01M85   LSPCTTTRNTVCQCEEGTFREEDSPEMCSSCRTGCPRGMVKVGDCTPWSDIECVHKE  127
YF01M86   LSPCTTTRNTVCQCEEGTFREEDSPEMCWWCRTGCPRGMVKVGDCTPWSDIECVHKE  127
YF01M87   LSPCTTTRNTVCQCEEGTFREEDSPEMCYYCRTGCPRGMVKVGDCTPWSDIECVHKE  127
```

**FIG. 4**

**FIG. 5**

**FIG. 6**

**FIG. 7**

**FIG. 8**

**FIG. 9**

1 PBS
2 YF01 3mg/kg
3 YF01 15mg/kg
4 YF01M3 3mg/kg
5 YF01M3G1m 3mg/kg
6 YF01M3G4 3mg/kg

**FIG. 10**

**FIG. 11**

**FIG. 12**

## INTERNATIONAL SEARCH REPORT

| International application No. |
|---|
| **PCT/CN2024/072443** |

**A. CLASSIFICATION OF SUBJECT MATTER**

C07K19/00(2006.01)i; C12N15/63(2006.01)i; C12N5/10(2006.01)i; C12N15/11(2006.01)i; A61K38/16(2006.01)i; A61K48/00(2006.01)i; A61P9/10(2006.01)i

According to International Patent Classification (IPC) or to both national classification and IPC

**B. FIELDS SEARCHED**

Minimum documentation searched (classification system followed by classification symbols)

IPC: C07K, C12N, A61K, A61P

Documentation searched other than minimum documentation to the extent that such documents are included in the fields searched

Electronic data base consulted during the international search (name of data base and, where practicable, search terms used)

CNABS; CNTXT; DWPI; WPABS; ENTXT; OETXT; VEN; CNKI; PubMed; WEB OF SCIENCE and keywords: 死亡受体5, TRAIL细胞表面受体2, 肿瘤坏死因子相关凋亡诱导配体, 半衰期, 融合蛋白, 心肌梗死, 心梗, 脑梗, death receptor, DR5, sDR5, TNF-related apoptosis-inducing ligand, TRAIL, half-life, fusion protein, myocardial infarction, MI, brain stem; 中国专利生物序列检索系统, China Patent Biological Sequence Search System; Genbank和检索的序列: SEQ ID NOs: 1, 24, Genbank and searched sequences: SEQ ID NOs: 1 and 24.

**C. DOCUMENTS CONSIDERED TO BE RELEVANT**

| Category* | Citation of document, with indication, where appropriate, of the relevant passages | Relevant to claim No. |
|---|---|---|
| A | CN 105061604 A (HENAN UNIVERSITY) 18 November 2015 (2015-11-18) see claims 1-10 | 1-43, 47-49 (all in part) |
| A | CN 102884083 A (NOVARTIS AG) 16 January 2013 (2013-01-16) see claims 1-31 | 1-43, 47-49 (all in part) |
| A | CN 102949708 A (HENAN UNIVERSITY) 06 March 2013 (2013-03-06) see claims 1-5 | 1-43, 47-49 (all in part) |
| A | EP 1788086 A1 (HUMAN GENOME SCIENCES INC.) 23 May 2007 (2007-05-23) see claims 1-33 | 1-43, 47-49 (all in part) |
| A | CN 108997503 A (AMSHENN BIOTECH, INC.) 14 December 2018 (2018-12-14) see claims 1-12 | 1-43, 47-49 (all in part) |
| A | CN 101300273 A (AMGEN INC.) 05 November 2008 (2008-11-05) see claims 1-62 | 1-43, 47-49 (all in part) |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **16 April 2024** | **30 April 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/072443** |

| **Box No. I**      **Nucleotide and/or amino acid sequence(s) (Continuation of item 1.c of the first sheet)** |
| --- |

1. With regard to any nucleotide and/or amino acid sequence disclosed in the international application, the international search was carried out on the basis of a sequence listing:

     a.   ☑   forming part of the international application as filed.

     b.   ☐   furnished subsequent to the international filing date for the purposes of international search (Rule 13*ter*.1(a)),

         ☐   accompanied by a statement to the effect that the sequence listing does not go beyond the disclosure in the international application as filed.

2. ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2024/072443** |

---

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **44-46, 50-53**
   because they relate to subject matter not required to be searched by this Authority, namely:

   PCT Rule 39.1(iv) – methods for treatment of the human or animal body by surgery or therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

Invention 1: Claims 1-43 and 47-49 (all in part) relate to a death receptor 5 (DR5) domain variant, an amino acid sequence thereof being SEQ ID NO: 1; and a corresponding fusion protein, nucleic acid molecule, vector, cell, preparation method, pharmaceutical composition and pharmaceutical use.

Invention 2: Claims 1-43 and 47-49 (all in part) relate to a death receptor 5 (DR5) domain variant, an amino acid sequence thereof being SEQ ID NO: 2; and a corresponding fusion protein, nucleic acid molecule, vector, cell, preparation method, pharmaceutical composition and pharmaceutical use.

Inventions 3-23: Claims 1-43 and 47-49 (all in part) relate to death receptor 5 (DR5) domain variants, amino acid sequences thereof being SEQ ID NOs: 3-23; and corresponding fusion proteins, nucleic acid molecules, vectors, cells, preparation methods, pharmaceutical compositions and pharmaceutical uses.

The same or corresponding technical feature of the 23 inventions is a "DR5 domain variant". This technical feature has been disclosed in the prior art (see, for example, CN 102884083 A, publication date: 16 January 2013). Therefore, the same or corresponding technical feature among inventions 1-23 does not make a contribution over the prior art, and cannot be considered to be a special technical feature (PCT Rule 13.2). Therefore, the present application does not comply with the requirement of unity of invention (PCT Rule 13.1).

---

Form PCT/ISA/210 (continuation of first sheet) (July 2022)

## INTERNATIONAL SEARCH REPORT

| International application No. |
| --- |
| **PCT/CN2024/072443** |

**Box No. III**      **Observations where unity of invention is lacking (Continuation of item 3 of first sheet)**

1. ☐   As all required additional search fees were timely paid by the applicant, this international search report covers all searchable claims.

2. ☐   As all searchable claims could be searched without effort justifying additional fees, this Authority did not invite payment of additional fees.

3. ☐   As only some of the required additional search fees were timely paid by the applicant, this international search report covers only those claims for which fees were paid, specifically claims Nos.:

4. ☑   No required additional search fees were timely paid by the applicant. Consequently, this international search report is restricted to the invention first mentioned in the claims; it is covered by claims Nos.: **Invention 1: Claims 1-43 and 47-49 (all in part)**

**Remark on Protest**     ☐   The additional search fees were accompanied by the applicant's protest and, where applicable, the payment of a protest fee.

                                    ☐   The additional search fees were accompanied by the applicant's protest but the applicable protest fee was not paid within the time limit specified in the invitation.

                                    ☐   No protest accompanied the payment of additional search fees.

Form PCT/ISA/210 (continuation of first sheet) (July 2022)

**INTERNATIONAL SEARCH REPORT**
Information on patent family members

| International application No. |
| --- |
| **PCT/CN2024/072443** |

| Patent document cited in search report | | | Publication date (day/month/year) | Patent family member(s) | | | Publication date (day/month/year) |
| --- | --- | --- | --- | --- | --- | --- | --- |
| CN | 105061604 | A | 18 November 2015 | WO | 2017028558 | A1 | 23 February 2017 |
| | | | | US | 2018161450 | A1 | 14 June 2018 |
| | | | | US | 10918739 | B2 | 16 February 2021 |
| CN | 102884083 | A | 16 January 2013 | GEP | 20146176 | B | 10 October 2014 |
| | | | | EA | 201201114 | A1 | 29 March 2013 |
| | | | | UA | 106900 | C2 | 27 October 2014 |
| | | | | MA | 34053 | B1 | 05 March 2013 |
| | | | | CL | 2012002217 | A1 | 31 January 2014 |
| | | | | MX | 2012009328 | A | 30 November 2012 |
| | | | | US | 2011318366 | A1 | 29 December 2011 |
| | | | | US | 9120855 | B2 | 01 September 2015 |
| | | | | UY | 33222 | A | 30 September 2011 |
| | | | | US | 2015259426 | A1 | 17 September 2015 |
| | | | | GT | 201200236 | A | 20 May 2015 |
| | | | | TW | 201130513 | A | 16 September 2011 |
| | | | | TWI | 480051 | B | 11 April 2015 |
| | | | | ZA | 201205864 | B | 29 May 2013 |
| | | | | KR | 20130031241 | A | 28 March 2013 |
| | | | | CR | 20120415 | A | 09 January 2013 |
| | | | | MY | 156337 | A | 15 February 2016 |
| | | | | SG | 183210 | A1 | 27 September 2012 |
| | | | | NI | 201200134 | A | 30 October 2012 |
| | | | | JP | 2013519364 | A | 30 May 2013 |
| | | | | AR | 080158 | A1 | 21 March 2012 |
| | | | | BR | 112012019924 | A2 | 02 May 2017 |
| | | | | AU | 2011214355 | A1 | 30 August 2012 |
| | | | | AU | 2011214355 | B2 | 22 May 2014 |
| | | | | EP | 2534176 | A1 | 19 December 2012 |
| | | | | CU | 20120116 | A7 | 28 February 2014 |
| | | | | CU | 24133 | B1 | 27 November 2015 |
| | | | | PE | 20121703 | A1 | 11 December 2012 |
| | | | | WO | 2011098520 | A1 | 18 August 2011 |
| | | | | IL | 221289 | A0 | 31 October 2012 |
| | | | | CO | 6561836 | A2 | 15 November 2012 |
| | | | | TN | 2012000393 | A1 | 30 January 2014 |
| | | | | CA | 2789251 | A1 | 18 August 2011 |
| | | | | NZ | 601582 | A | 25 July 2014 |
| CN | 102949708 | A | 06 March 2013 | WO | 2014071670 | A1 | 15 May 2014 |
| EP | 1788086 | A1 | 23 May 2007 | None | | | |
| CN | 108997503 | A | 14 December 2018 | WO | 2018223764 | A1 | 13 December 2018 |
| CN | 101300273 | A | 05 November 2008 | UA | 97096 | C2 | 10 January 2012 |
| | | | | ZA | 200802575 | A | 28 January 2009 |

Form PCT/ISA/210 (patent family annex) (July 2022)

## EP 4 653 468 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015036583 A

**Non-patent literature cited in the description**

- **W. R. PEARSON** ; **D. J. LIPMAN**. Improved Tool for Biological Sequence Comparison. *Proc. Natl. Acad. Sci. USA*, 1988, vol. 85, 2444-2448
- **D. J. LIPMAN** ; **W. R. PEARSON**. Fast and Sensitive Protein Similarity Search. *Science*, 1989, vol. 227, 1435-1441
- **S. ALTSCHUL** ; **W. GISH** ; **W. MILLER** ; **E. W. MYERS** ; **D. LIPMAN**. A Basic Local Alignment Search Tool. *Molecular Biology*, 1990, vol. 215, 403-410
- Molecular Cloning. **SAMBROOK et al.** A Laboratory Manual. Cold Spring Harbor Laboratory Press, 1989
- **AUSUBE et al.** Current Protocols in Molecular Biology. Greene Publishing and Wiley-Interscience, 1993
- Remington: The Science and Practice of Pharmacy. Gennaro, Mack Publishing Co., 1995